# EUROPEAN PATENT APPLICATION

(11) **EP 1 647 596 A2**
(43) Date of publication of application: **19.04.2006**
(21) Application number: 04747959.7
(22) Date of filing: 20.07.2004
(51) Int. Cl.: C12N 15/13, C07K 16/18, C12N 5/10, A61K 39/395, C12P 21/02, C12P 21/08

(54) **MONOCLONAL ANTIBODY AGAINST PLATELET MEMBRANE GLYCOPROTEIN VI**

(30) Priority: 18.07.2003 JP 2003199192
(71) Applicant: MOCHIDA PHARMACEUTICAL CO., LTD., Shinjuku-ku Tokyo 160-8515 (JP)
(72) Inventor: TAKAYAMA, Hiroshi, Ibaraki-shi, Osaka 5670895 (JP); SHIRAKAWA, Kamon, Mochida Pharmaceutical Co., Ltd., Tokyo 1608515 (JP); YAMAKAWA, Tooru, Mochida Pharmaceutical Co., Ltd., Tokyo 1608515 (JP); KAWAHARA, T., Mochida Pharmaceutical Co., Ltd., Tokyo 1608515 (JP)
(74) Representative: Helbing, Jörg
(86) International application number: PCT/JP2004/010596
(87) International publication number: WO 2005/007800

(57) **Abstract**

The present invention provides a human antibody or an active-fragment thereof that specifically binds to human platelet membrane glycoprotein VI and does not induce a human platelet aggregation independently; a cell that produces the antibody or its active-fragment; a pharmaceutical composition that comprises the antibody or its active-fragment as an active ingredient, and so on. The above-mentioned cell can be obtained for example, as follows: a peripheral-blood-lymphocyte of the human that produces an autologous antibody to GPVI is activated by in vitro immunization under specific conditions; a hybridoma with mouse myeloma cell is prepared; and then the hybridoma that produces a monoclonal antibody, which has a binding capacity to GPVI and has an activity that suppresses collagen-mediated agglutinability of the human platelet is selected.

## Description

### TECHNICAL FIELD

The present invention relates to an antibody to human platelet membrane glycoprotein VI (hereinafter, sometimes abbreviated as GPVI) and a cell that produces the antibody.

### BACKGROUND ART

The platelet plays a very important role in the blood clotting and the biophylaxis, and its concerning in various clinical condition is being elucidated from the physiological role. In particular, it is remarkable about the function that the platelet forms a hemostatic plug. For example, when the vascular endothelial cell suffers damage, the collagen that is the major matrix protein of the subcutaneous vascular endothelium is exposed and the platelet adheres thereto. Next, the platelet is activated by the signal from the collagen and when finally, the platelet agglutinates through the fibrinogen. Then, since this fact causes morbidity such as thromboembolic disease depending on the situation, it is remarkable as a target for therapy.

In the past, for the purpose of the treatment and the prevention of the thrombosis based on the platelet aggregation, an anti-platelet agent such as aspirin, ticlopidine, GPIIb/IIIa antagonist and the like has been used. However, a lot of problems are pointed out from the aspect of the effectivity and the side effects such as the bleeding. Therefore, the excellent platelet inhibitor having the enough safety and the sure and appropriate function without the above-mentioned problems is desired to arrive.

GPVI that is present on the platelet membrane is the collagen receptor of the platelet, and it has been elucidated that the GPVI plays a central role for an activation of the platelet by collagen stimulation (see, Non-patent document 1: Hiroshi Takayama, The Japanese Journal of Thrombosis and Hemostasis, 2003, volume 14, No. 2, pp. 75-81). That is, Sugiyama et al. has been reported that the membrane protein of 62kDa is specifically deleted in the platelet of the patient with autoimmune thrombocytopenia and the platelet aggregation by the collagen cannot be detected (see, Non-patent document 2: Tateo Sugiyama and five other members, Blood (USA), 1987, volume 69, No. 6, pp. 1712-1720), and further that the protein that has been deleted in the platelet of the patient is GPVI and the Fab fragment of the antibody purified from the serum of the patient suppresses a collagen-induced platelet aggregation (see, Non-patent document 2, and Non-patent document 3: Masaaki Moroi and three other members, The Journal of Clinical Investigation (USA), 1989, volume 84, No. 5, pp. 1440-1445).

So far, Sugiyama et al. (see, Non-patent document 2) and Takahashi et al. (see, Non-patent document 4) have been reported about the anti-human GPVI autologous antibody derived from the patient with autoimmune disease. However, since, according to the report by Sugiyama et al., there has a function that induces a platelet aggregation in the anti-human GPVI autologous antibody purified from the plasma of the patient, it cannot be applied for medicaments immediately. Non-patent document 4 (Hoyu Takahashi and one other member, American Journal of Hematology (USA), 2001, volume 67, No. 4, pp. 262-267) describes that an autologous antibody to the protein of ca. 62kDa that is speculated to be GPVI is present, and that this antibody induces a platelet aggregation. In addition, to apply these axxti-GPVI antibodies derived from the patient clinically as a medicament, the antibody with high safety must be produced in quantities in the stable quality. However, the method of producing industrially is not yet established.

The anti-GPVI antibody, which is prepared by the present, includes a monoclonal rat antibody to mouse GPVI (see, Patent document 1: Publication number 1228768 of the European Patent Application) and a monoclonal mouse antibody to human GPVI (see, Patent document 2: Publication number 01/00810 of International Patent Application and Patent document 3: Publication number 02/080968 of International Patent Application, Thromb Haemost. 2003 Jun; 89(6): 996-1003). Since these antibodies are an antibody derived from non-human animal and when those are administered to human, side effects due to high immunogenicity (sometimes referred to as "antigenicity") are feared, it is inappropriate that the antibodies are directly administered to human, and it is purely desired that the antibody to be administered to human is a human antibody derived from the human.

Also, a human single-stranded antibody that recognizes the human GPVI (scFv: single chain Fv) has been prepared using the phage display method and so on (see, Patent documents 2 and 3, and Non-patent document 5: Peter A Smethurst and 15 other members, Blood (USA), 2002, volume 100, No. 11, p. 474a). These single-stranded antibodies are the antibody that combines VH and VL of the human antibody by the peptide linker and has a variable region derived from the human. However, compared with normal immunoglobulin that the cell produces, it generally possesses a low affinity to an antigen thereof and is short in the half-life in the vivo, too.

A method of preparing a humanized antibody by transplant of the complementary determining region (CDR) is publicly known. However, since the amino acid sequence of CDR and, in many cases, a portion of the framework region (FR) are derived from a non-human animal antibody, and all of the amino acid sequence of the antibody is not derived from the human, the possibility that an antibody to the antibody that has been administered is in vivo produced is pointed out. In addition, it cannot be said that it is effective and safe as a medicament from the aspect of the immunogenicity. There are plural reports about the preparation of a human antibody, but in the method of either report, there has many problems. Further, since, at the present time, these are not perceived as a generalized method applicable to all antibodies, it is generally difficult to acquire a human antibody with high titer.

### DISCLOSURE OF THE INVENTION

In the context of requirement for a medicament that has a high safety, an excellent efficacy, and a usability as an anti-platelet agent as described above, an anti-GPVI antibody purely derived from the human is desired.

The present invention intends to provide a novel antibody that specifically binds to GPVI, which is the glycoprotein that exists on the human platelet membrane, preferably a monoclonal antibody. Particularly, there is provided the anti-GPVI human antibody that is applicable to the human, has efficacy and no problem in the aspect of side effects, and is purely derived from the human. Also, the invention provides the antibody that specifically binds to human GPVI and comprises a novel CDR sequence.

Moreover, the cell that produces these antibodies, specifically, a specific hybridoma is provided.

To solve the above-mentioned problems, the inventors have conceived of acquisition of a human antibody that effectively suppresses the GPVI-mediated platelet aggregation using the lymphocyte of the human who produces an autologous antibody to GPVI as a starting material. Based on the idea, the present inventors have made extensive investigations and have succeeded to obtain the hybridomas that produce the antibody having a binding capacity to GPVI and an activity for suppressing the platelet aggregation by collagen from plural hybridomas when lymphocytes from peripheral blood were activated by in vitro immunization under specific conditions and hybridomas with mouse myeloma cells were prepared. As a result of further investigation, the inventors succeeded in isolating the clone and obtaining a gene encoding the antibody. In addition, it was found that the amino acid sequence of CDR of the antibody is a novel one. Moreover, the inventors have completed the present invention by preparing a recombinant antibody using genetically engineered techniques.

Herein throughout the specification, the antibody that is produced by hybridoma such as the clone #2-6 is designated as #2-6 antibody, and further the genetically engineered antibody from the gene encoding the antibody that is produced by the hybridoma is designated as R#2-6 antibody.

The first embodiment of the present invention is a human antibody that specifically binds to human GPVI, preferably a monoclonal antibody (hereinafter, referred to as anti-human GPVI antibody and human GPVI monoclonal human antibody, respectively), or an active fragment thereof, more preferably an antibody that does not induce a human platelet aggregation solely, or an active fragment thereof. Specifically, it includes as follows:
(1) A human antibody or its active fragment that specifically binds to human GPVI and suppresses a collagen-mediated human platelet aggregation by administering it to the living body, wherein preferably, for a platelet isolated from the blood after administering the antibody or the active fragment to the living body, agglutinability induced by collagen is impaired or not detected compared with normal platelet;
(2) A human antibody or its active fragment that has any one or more of functions for specifically inhibiting the binding between human GPVI and collagen on the platelet by specifically binding to GPVI, disappearing a functional GPVI on the platelet, or suppressing a collagen-mediated human platelet aggregation by preliminarily contacting with human platelet, that is, depressing or deleting an agglutinability of platelet responsive to collagen;
(3) The antibody or its active fragment of the above (1) to (2), which suppresses a collagen-mediated human platelet aggregation by internalizing human GPVI on the platelet into the platelet cell, or cleaving human GPVI.
   With regard to the antibody of the above (1) to (3), preferred are the antibody that does not induce a human platelet aggregation solely, and/or that does not induce a thrombocytopenia when administering to the living body. Preferred examples are the antibodies that are produced by hybridoma clones #2-6 or #2-4, or a humanized IgG, preferably a humanized IgG4 antibody genetically engineered. Also, the antibody of the present invention is an antibody having a dissociation constant (Kd value) between human GPVI and the antibody of preferably equal to or less than 100 nM, more preferably, equal to or less than 50 nM. The active fragment of the antibody of the present invention is, for example, a peptide comprising Fab (Fragment of antigen binding), Fab', F(ab')₂, single-stranded antibody (scFv), disulfide-stabilizing antibody (dsFv), CDR and so on, in so far as having a binding ability to GPVI.
   Also, specifically,
(4) It includes a human antibody or its active fragment that specifically binds to human GPVI and suppresses a collagen-mediated human platelet aggregation, but not an aggregation by thrombin, and further does not induce a human platelet aggregation solely. The antibody is an antibody or its active fragment that in the concentration or the dosage equivalent to those, which suppress a collagen-mediated human platelet aggregation, preferably 10-fold, more preferably 100-fold, further preferably 1000-fold, does not significantly induce a human platelet aggregation solely.
   Herein, among the antibodies of the above (1) or (4), an antibody that inhibits a binding between human GPVI and collagen is one having a dissociation constant (Kd value) of preferably equal to or less than 10 nM, more preferably equal to or less than 1 nM, further preferably equal to or less than 0.1 nM.
   The antibody of the present invention is not always limited to the specific clone, and the antibody having a similar function to that of the preferred examples of the present invention (#2-6, #2-4, R#2-6 or the R#2-4 antibodies and so on) is encompassed within the scope of the present invention. The existence or non-existence of the function of the antibody of the present invention can be confirmed by the method shown in Examples or the publicly known method.
   In addition, an antibody, wherein the binding site or the epitope on GPVI is identical or at least partially common to those of the preferred antibody of the present invention, e.g. an antibody that competes each other when binding to GPVI is included within the scope of the present invention. The existence or non-existence of the common characteristic of the binding-site with the antibody of the present invention can be confirmed according to the method described in Examples or by the publicly known method.
   The second embodiment of the present invention is an anti-human GPVI antibody comprising a novel amino acid sequence of CDR or variable region, preferably a monoclonal antibody. Specifically, it includes:
(5) An anti-human GPVI antibody or an active fragment thereof, wherein at least three sets of CDR of either H-chain or L-chain of antibody, preferably six sets of CDR of both H-chain and L-chain of antibody comprise an amino acid sequence of CDR of the antibody produced by any hybridoma selected from a group consisting of the clones listed in Table 4, preferably clones #2-6 and #2-4 as a corresponding amino acid sequence of respective CDRs;
(6) An antibody or an active fragment thereof, which comprises the amino acid sequences of SEQ ID NO: 47, SEQ ID NO: 48, SEQ ID NO: 49, SEQ ID NO: 98, SEQ ID NO: 99, and SEQ ID NO: 100 in VH CDR1, VH CDR2, VHCDR3, VLCDR1, VLCDR2 and VLCDR3, respectively, or the amino acid sequences of SEQ ID NO: 7, SEQ ID NO: 8, SEQ ID NO: 9, SEQ ID NO: 10, SEQ ID NO: 11, and SEQ ID NO: 12 in VH CDR1, VH CDR2, VHCDR3, VLCDR1, VLCDR2 and VLCDR3, respectively;
(7) A human GPVI antibody or an active fragment thereof, wherein at least a variable region of H-chain or L-chain of antibody, preferably variable regions of both H-chain and L-chain of antibody comprise an amino acid sequence of the variable region having the antibody produced by any hybridoma selected from a group consisting of the clones listed in Table 4, preferably clones #2-6 and #2-4 as a corresponding amino acid sequence of the respective variable regions;
(8) An antibody or an active fragment thereof, which comprises the amino acid sequences of SEQ ID NO: 143 and SEQ ID NO: 144 within the H-chain variable region and the L-chain variable region, respectively, or an antibody or an active fragment thereof, which comprises the amino acid sequences of SEQ ID NO: 15 and SEQ ID NO: 16 within the H-chain variable region and the L-chain variable region, respectively;
(9) A monoclonal antibody, which is produced by the #2-6 cell or the #2-4 cell, or an active fragment thereof.
   The third embodiment of the present invention is a cell which produces the antibody of the first or the second embodiment. Specifically, it includes:
(10) A transformant, which produces any antibody described in the above (1) or (9);
(11) The cell of the above (10), which is #2-6 or #2-4 cell.
   The fourth embodiment of the present invention is a polynucleotide or nucleic acid, which comprises a base sequence encoding three sets of CDR, preferably a variable region in at least, either H-chain or L-chain of the antibody or an active fragment thereof of the first or the second embodiment. Specifically, it is the antibody or an active fragment thereof of the first or the second embodiment, and includes:
(12) A polynucleotide, which comprises, as the base sequence encoding at least three sets of CDR of either H-chain or L-chain of antibody, preferably six sets of CDR of both H-chain and L-chain of antibody, in a gene encoding the antibody of any hybridoma selected from a group consisting of the clones listed in Table 4, preferably clones #2-6 and #2-4, a corresponding base sequence encoding the respective CDRs;
(13) A polynucleotide, which comprises the base sequence of SEQ ID NO: 147 encoding VH CDR1, the base sequence of SEQ ID NO: 148 encoding VH CDR2, the base sequence of SEQ ID NO: 149 encoding VHCDR3, the base sequence of SEQ ID NO: 150 encoding VL CDR1, the base sequence of SEQ ID NO: 151 encoding VL CDR2, and the base sequence of SEQ ID NO: 152 encoding VL CDR3, respectively, or the base sequence of SEQ ID NO: 23 encoding VH CDR1, the base sequence of SEQ ID NO: 24 encoding VH CDR2, the base sequence of SEQ ID NO: 25 encoding VHCDR3, the base sequence of SEQ ID NO: 26 encoding VL CDR1, the base sequence of SEQ ID NO: 27 encoding VL CDR2, and the base sequence of SEQ ID NO: 28 encoding VL CDR3, respectively;
(14) A polynucleotide, which comprises, as at least a variable region of H-chain or L-chain of antibody, preferably variable regions of both H-chain and L-chain of antibody, in a gene encoding the antibody of any hybridoma selected from a group consisting of the clones listed in Table 4, preferably clones #2-6 and #2-4, a corresponding base sequence encoding the respective variable regions;
(15) A polynucleotide, which comprises the base sequences of SEQ ID NO: 145 encoding the H-chain variable region and the base sequence of SEQ ID NO: 146 encoding the L-chain variable region, respectively, or a polynucleotide, which comprises the base sequences of SEQ ID NO: 31 encoding the H-chain variable region and the base sequence of SEQ ID NO: 32 encoding the L-chain variable region, respectively.
   The fifth embodiment of the present invention is a method of manufacturing the antibody of the first or second embodiment. Specifically, it is the method of manufacturing the antibody of the first or second embodiment, and includes:
(16) A method of manufacturing, which comprises a process for culturing the cells of the above (10) and a process for collecting a monoclonal antibody produced by the cells;
(17) A method of manufacturing, which comprises a process for culturing the cells of the above (11) and a process for collecting a monoclonal antibody produced by the cells; and
(18) A method of manufacturing, which comprises a process for using any of the polynucleotide of the fourth embodiment, an expression vector comprising the same, and a cell comprising the polynucleotide or the expression vector.

The sixth embodiment of the present invention relates to a pharmaceutical composition comprising the antibody of the first or the second embodiment of the present invention as an active ingredient, preferably a pharmaceutical composition for prevention and/or treatment of thrombotic, embolic or arteriosclerosis diseases.

The seventh embodiment of the present invention is a method of diagnosing diseases by detection or quantification of GPVI in the sample using the antibody of the first or the second embodiment of the present invention, preferably a method of diagnosing diseases associated with abnormality of the blood clotting.

The eighth embodiment of the present invention is a method of manufacturing another class of human antibody such as IgG antibody, particularly human IgG4 antibody or a polynucleotide thereof by recombining a recombinant human antibody, particularly recombinant human-human chimeric antibody, specifically a human antibody such as IgM antibody with genetically engineered techniques, and comprises a process for recombining, for example, a polynucleotide encoding the antibody that is produced by the hybridoma (e.g. human IgM) and a polynucleotide encoding the publicly known human antibody (e.g. IgG4 antibody) by genetically engineered techniques. The procedures include the PCR method using the hybridoma mRNA and/or the genomic DNA as a template, specifically the method described in EXAMPLE 9, preferably EXAMPLE 10. In EXAMPLE 10, a polynucleotide encoding a target antibody can easily be manufactured by amplifying multiple exons with PCR using genomic DNA as a template, and mixing multiple PCR amplified product (corresponding to four kinds of IgG) to perform PCR simultaneously.

### BRIEF DESCRIPTION OF THE DRAWINGS

Figure 1 is the flow chart which shows the construction of pCAGGS-GPVI-Fc, the GPVI-Fc expressing plasmid.
Figure 2 is the flow chart which shows the construction of pYNG-GPVI-Fc which is the clone of the transfer vector to prepare a recombinant virus.
Figure 3 is the graph which shows a result of measuring the binding activities of 18 kinds of anti-GPVI monoclonal human antibodies by the ELISA method based on the reactivity with GPVI-Fc.
Figure 4 is the graph which shows that the recombinant anti-GPVI human antibody (R#2-4 and R#2-6) binds to a platelet prepared from the human peripheral blood.
Figure 5 is the graph which shows a binding affinity of various humanized IgG antibody to GPVI to GPVI-hFc.

### BEST MODE FOR CARRYING OUT THE INVENTION

### (Constitution)

The antibody of the first embodiment of the present invention specifically recognizes GPVI, the membrane glycoprotein which exists on the human platelet. In addition, GPVI that is recognized by the antibody of the present invention is not always limited to the one that is present on the platelet. The antibody can also recognize, for example, GPVI on megakaryocyte. The present invention is described in detail below.

The antibody of the present invention is a monoclonal antibody. A method of preparing the monoclonal antibody is not limited to a specific method. For example, the monoclonal antibody may be any one of the one that is produced by hybridoma, the one that is produced by a recombinant cell, in which a gene encoding the antibody is incorporated, or the one that is produced by a cell transformed by Epstein-Barr virus (EBV).

The human antibody is an antibody, wherein both the whole variable region and the whole constant region consist of the amino acid sequences derived from human. A method of preparing the human antibody of the present invention is not limited to a specific method. For example, the human antibody may be any of the one that is produced by human-human hybridoma, the one that is produced by a transgenic animal, the one that is produced by a recombinant cell, in which a gene encoding the human antibody is incorporated, the one that is produced by a human cell transformed by EBV, or the one that is produced by a hybridoma prepared using human lymphocyte that produces an autologous antibody.

The antibody of the present invention is an antibody that specifically binds to human GPVI. In the antibody of the present invention, the dissociation constant (the Kd value) of human GPVI with the antibody is preferably less or equal to 100 nM, more preferably less or equal to 50nM. A method of measuring the dissociation constant of human GPVI with the antibody is not limited to a specific method, and a conventional manner can be done. For example, the dissociation constant can be determined by the protein interaction analyzer such as BIACORE3000 using GPVI-Fc immobilized on the chip. Specifically, it is shown in EXAMPLE 7.

The antibody of the present invention has the activity that suppresses the human platelet aggregation by the collagen. Herein, the platelet aggregation activity can be measured by the publicly known method. For example, it can be measured by calculating an aggregation rate using a light transmission with the platelet aggregation analyzer as an index, and in general, may be represented by the aggregation rate at the point that exhibits a maximum light transmission (hereinafter, sometimes referred to as the maximum aggregation rate). In the method described in EXAMPLE 6 below, the antibody of the present invention at the concentration preferably equal to or less than 10 µg/mL, more preferably equal to or less than 1 µg/mL, further preferably equal to or less than 0.1 µg/mL, makes the maximum aggregation rate decrease by preferably equal to or less than 50%, more preferably equal to or less than 30%, further preferably equal to or less than 20%, most preferably equal to or less than 10% of the control. The method of measuring the suppression of human platelet aggregation by collagen is not limited to the above-mentioned method and can also be done by the other conventional method. Further, in the method described in EXAMPLE 6, regardless of existence or non-existence for the suppressing activity of the collagen-mediated human platelet aggregation, that is, the direct function, preferred is an antibody having an activity that indirectly suppresses a collagen-induced human platelet aggregation by attenuating or deleting the ability for the human platelet to aggregate, replying to the collagen. For example, in the method described in EXAMPLE 15, the antibody of the present invention at the concentration preferably equal to or less than 30 µg/mL, more preferably equal to or less than 10 µg/mL, further preferably equal to or less than 1 µg/mL, makes the maximum aggregation rate decrease by preferably equal to or less than 30%, preferably equal to or less than 10%, morepreferably equal to or less than 5% of the control.

Preferably, the antibody of the present invention does not suppress an aggregation by the material other than collagen that induces a platelet aggregation, e.g. thrombin. In the method described in EXAMPLE 6 below, when the antibody of the present invention is preferably at least 0.1 µg/mL, more preferably at least 1 µg/mL, further preferably at least 10 µg/mL, especially preferably at least 100 µg/mL, the maximum aggregation rate is preferably at least 80%, more preferably at least 85%, further preferably 90%, most preferably at least 95% of the control. A method of measuring the suppression of the human platelet aggregation by the material that induces a platelet aggregation mediated by the material other than collagen is not limited to the above-mentioned method and can also be done by the other conventional method.

The antibody of the present invention does not enhance or induce an human platelet aggregation by the antibody solely, that is, without the substance that induces a platelet aggregation. In the method described in EXAMPLE 6 below, when the antibody of the present invention is preferably at least 0.1 µg/mL, more preferably at least 1 µg/mL, further preferably at least 10 µg/mL, especially preferably at least 100 µg/mL, the maximum aggregation rate is preferably equal to or less than 20%, more preferably equal to or less than 10%. A method of measuring a human platelet aggregation by the sole antibody is not always limited and can also be done by the other conventional method. In addition, for example, using whole blood, preferably whole blood treated with anti-thrombin agent (Argatroban etc.), an effect on the platelet can be indirectly evaluated. An example was shown in EXAMPLE 14.

Since most of the antibodies to the human GPVI, including the above-mentioned human autologous antibody, that have been reported until now, possess, in vitro, an activity that activates a platelet by the antibody itself, and/or an activity that induces or enhance a platelet aggregation, when they are administered to the living body, the possibility to cause a thrombocytopenia may be considered. In the form of the Fab fragment and so on, the one that does not induce a platelet aggregation is also reported, meanwhile in the living body, a possibility that Fab behaves in a similar manner to IgG by cross-linking or aggregating from any cause cannot fully be denied. Therefore, in a intact antibody molecule, but not an active fragment of the antibody, for example, IgG form, an anti-GPVI antibody that does not exhibit the above-mentioned activity, or has a low activity is preferred.

Also, for behavior and stability in the living body, the antibody molecule which is the natural form, e.g. IgG, is superior. Generally, a half-life of the IgG in the blood is much longer than that of the fragment such as Fab. Thus especially, for chronic diseases such as thrombosis and the like, or clinical conditions that necessitate an antibody administration over long period, a molecular form having a long half-life in the blood, particularly IgG is desirable.

The antibody of the present invention may specifically inhibit the binding of GPVI on the platelet to collagen, and is, in the method described in EXAMPLE 7 below, an antibody that inhibits the binding of GPVI and collagen by 50% at the concentration preferably equal to or less than 100 µg/mL, more preferably equal to or less than 10 µg/mL, further preferably equal to or less than 1 µg/mL, especially preferably equal to or less than 0.1 µg/mL A method of measuring the binding of collagen and GPVI is not limited to a specific method and can also be done by the other conventional method.

The antibody of the present invention suppresses a collagen-induced platelet aggregation by being administered in vivo. For the mechanism of suppressing a collagen-induced platelet aggregation by the antibody of the present invention, it is considered that (i) the antibody of the present invention inhibits the binding of collagen exposed by vascular endothelial cell damage and GPVI presented on the platelet; (ii) the antibody of the present invention preliminarily binds to GPVI on the surface of the platelet to provide the condition not to bind to collagen; (iii) the antibody of the present invention internalizes GPVI by binding to GPVI on the surface of platelet and/or megakaryocyte so that GPVI is disappeared from the platelet surface; or (iv) using an activity that the antibody of the present invention has, GPVI on the surface of platelet and/or megakaryocyte is cleaved to make GPVI disappear from the platelet surface. Any mechanism of the human antibody of the present invention for suppressing a collagen-induced platelet aggregation may be selectable, but multiple mechanisms may be combined.

The second embodiment of the present invention is an anti-human GPVI monoclonal antibody, which comprises an amino acid sequence of novel CDR or variable region.

On the N-terminal end of heavy chain and light chain of antibody, variable region exists, and is designated heavy chain variable region (VH) and light chain variable region (VL), respectively. Within the variable region, complementarity determining region (CDR) is present, and assumes specificity for recognition of antigen. A portion except CDR in variable region has the role that maintains the structure of CDR and is called framework region (FR). On the C-terminal end of heavy chain and light chain of antibody, constant region exists, and is designated heavy chain constant region (CH) and light chain constant region (CL), respectively.

In the heavy chain variable region, three complementarity determining regions exist: the first complementarity determining region (CDR1), the second complementarity determining region (CDR2) and the third complementarity determining region (CDR3). These three complementarity determining regions in the heavy chain variable region collectively mean a heavy chain complementarity determining region. As is the case with the heavy chain, in the light chain variable region, three complementarity determining regions exist: the first complementarity determining region (CDR1), the second complementarity determining region (CDR2) and the third complementarity determining region (CDR3). These three complementarity determining regions in the light chain variable region collectively mean a light chain complementarity determining region.

Although CDR sequence of the antibody of the present invention is not always limited, preferred antibody is an antibody that comprises any one or more sequences among the amino acid sequence of SEQ ID NO: 47 as VH CDR1, the amino acid sequence of SEQ ID NO: 48 as VH CDR2, the amino acid sequence of SEQ ID NO: 49 as VH CDR3, the amino acid sequence of SEQ ID NO: 98 as VL CDR1, the amino acid sequence of SEQ ID NO: 99 as VL CDR2, and the amino acid sequence of SEQ ID NO: 100 as VL CDR3, preferably three sequences from the heavy chain, more preferably all the sequences.

The amino acid sequence of VH and VL of the antibody of the present invention is not always limited, but preferred antibody is an antibody that comprises any one or more among the amino acid sequence of SEQ ID NO: 143 as VH or the amino acid sequence of SEQ ID NO: 144 as VL, or an antibody that comprises any one or more among the amino acid sequence of SEQ ID NO: 15 as VH or the amino acid sequence of SEQ ID NO: 16 as VL.

In addition, the antibody of the present invention is not always limited to that with the specific amino acid sequence, and within the range where there is virtually no influence on its activity and/or antigenecity, as for the amino acid sequence of the antibody of the present invention, for example, variable region, especially FR portion, addition, deletion, substitution and/or insertion of one to several amino acid residues are permissible.

The antibody of the present invention is an antibody that the constant region of the antibody consists of an amino acid sequence derived from preferably human antibody, more preferably human IgG, further preferably human IgG4.

The antibody of this invention is not always limited to the specific molecular species. The structure of the antibody, i.e. the immunoglobulin consists of heavy chain (H-chain) and light chain (L- chain) and is divided into five isotypes (IgG, IgA, IgM, IgD, IgE) based on the class of the heavy chain (γ, α, µ, δ, ε). Among them, IgG and IgA are divided, based on difference of the heavy chain (e.g., in the case of the human, γ1,γ2,γ3, γ4, α1, α2), into subclasses (e.g., in the case of the human, IgG1, IgG2, IgG3, IgG4, IgA1, IgA2). The light chain is classified into either κ or λ type. The class, the subtype or the isotype of the antibody of the present invention is not limited, and may be the one that is classified into either. Preferred isotype is IgG, and further preferably, the subclass is IgG4 in the point that there is no complement fixation.

The antibody of the present invention, as long as the antibody has the activity such as a binding ability to GPVI, may be the fragment or the part of the antibody. For example, Fab (fragment of antigen binding), Fab', (Fab')₂, single-stranded antibody (scFv), disulfide stabilizing antibody (dsFv), the peptide comprising CDR and so on are included.

In the third embodiment of the present invention, a cell that produces the antibody of the present invention is provided. Examples of such a cell include hybridoma, transformant, or a genetically engineered cell, in which the gene encoding the antibody of the present invention is introduced. The hybridoma which produces an antibody includes, specifically, the hybridoma, the #2-6 cell or #2-4 cell, which was prepared by using the lymphocyte of the peripheral blood collected from the human who produces an autologous antibody to GPVI. Also, by the present invention, the antibody that the cell of the above invention produces is provided. The antibody-producing cell is not limited to the specific cell. Preferred is the antibody that is produced by the hybridoma prepared using the lymphocyte of the peripheral blood collected from the human who produces an autologous antibody to GPVI, further preferably, the antibody that the #2-6 cell or the #2-4 cell produced, and a recombinant antibody prepared from said antibody by recombinant DNA techniques.

In the fourth embodiment of the present invention, a polynucleotide or nucleic acid encoding the antibody of the first embodiment and the second embodiment of the present invention is provided. The polynucleotide is not always limited as long as that encodes the amino acid sequence of the antibody of the present invention, and includes DNA and RNA.

The polynucleotide encoding CDR sequence of the antibody of the present invention is not always limited, but is a polynucleotide comprising preferably any one or more sequences among the base sequence of SEQ ID NO: 147 encoding the amino acid sequence as VH CDR1, the base sequence of SEQ ID NO: 148 encoding the amino acid sequence as VH CDR2, the base sequence of SEQ ID NO: 149 encoding the amino acid sequence as VH CDR3, the base sequence of SEQ ID NO: 150 encoding the amino acid sequence as VL CDR1, the base sequence of SEQ ID NO: 151 encoding the amino acid sequence as VL CDR2 or the base sequence of SEQ ID NO: 152 encoding the amino acid sequence as VL CDR3, more preferably three sequences of the H-chain, further preferably all sequences, or a polynucleotide comprising preferably any one or more sequences among the base sequence of SEQ ID NO: 23 encoding the amino acid sequence as VH CDR1, the base sequence of SEQ ID NO: 24 encoding the amino acid sequence as VH CDR2, the base sequence of SEQ ID NO: 25 encoding the amino acid sequence as VH CDR3, the base sequence of SEQ ID NO: 26 encoding the amino acid sequence as VL CDR1, the base sequence of SEQ ID NO: 27 encoding the amino acid sequence as VL CDR2 or the base sequence of SEQ ID NO: 28 encoding the amino acid sequence as VL CDR3, more preferably three sequences of the H-chain, further preferably all sequences.

The polynucleotide encoding the amino acid sequence of VH and VL of the antibody of the present invention is not always limited, but is a polynucleotide comprising preferably either the base sequence of SEQ ID NO: 145 encoding the amino acid sequence as VH or the base sequence of SEQ ID NO: 146 encoding the amino acid sequence as VL, more preferably both base sequences, or a polynucleotide comprising preferably either the base sequence of SEQ ID NO: 31 encoding the amino acid sequence as VH or the base sequence of SEQ ID NO: 32 encoding the amino acid sequence as VL, more preferably both base sequences.

The polynucleotide encoding the constant region of the antibody of the present invention comprises a base sequence derived from preferably human antibody, more preferably human IgG, further preferably human IgG4.

By transferring a gene comprising the nucleotide sequence of the antibody of the present invention into a cell, the cell that produces the antibody of the present invention can be produced. As the gene to be transferred, preferred is a gene comprising any one or more sequences among the base sequence of SEQ ID NO: 147 encoding the amino acid sequence as VH CDR1, the base sequence of SEQ ID NO: 148 encoding the amino acid sequence as VH CDR2, the base sequence of SEQ ID NO: 149 encoding the amino acid sequence as VH CDR3, the base sequence of SEQ ID NO: 150 encoding the amino acid sequence as VL CDR1, the base sequence of SEQ ID NO: 151 encoding the amino acid sequence as VL CDR2 or the base sequence of SEQ ID NO: 152 encoding the amino acid sequence as VL CDR3, or a gene comprising any one or more sequences among the base sequence of SEQ ID NO: 23 encoding the amino acid sequence as VH CDR1, the base sequence of SEQ ID NO: 24 encoding the amino acid sequence as VH CDR2, the base sequence of SEQ ID NO: 25 encoding the amino acid sequence as VH CDR3, the base sequence of SEQ ID NO: 26 encoding the amino acid sequence as VL CDR1, the base sequence of SEQ ID NO: 27 encoding the amino acid sequence as VL CDR2 or the base sequence of SEQ ID NO: 28 encoding the amino acid sequence as VL CDR3. Further, as the gene to be transferred, preferred is a gene comprising any one or more sequences of the base sequence of SEQ ID NO: 145 encoding the amino acid sequence as VH or the base sequence of SEQ ID NO: 146 encoding the amino acid sequence as VL, or a gene comprising any one or more sequences of the base sequence of SEQ ID NO: 31 encoding the amino acid sequence as VH or the base sequence of SEQ ID NO: 32 encoding the amino acid sequence as VL. Furthermore, as the gene to be transferred, preferred is a gene having the base sequence of SEQ ID NO: 145 encoding the amino acid sequence as VH and the base sequence of SEQ ID NO: 146 encoding the amino acid sequence as VL, or a gene having the base sequence of SEQ ID NO: 31 encoding the amino acid sequence as VH or the base sequence of SEQ ID NO: 32 encoding the amino acid sequence as VL; more preferably a gene comprising the base sequence, wherein the constant region encodes the amino acid sequence derived from human antibody.

### (Manufacturing method)

As the fifth embodiment of the present invention, a method of producing the antibody is provided. A method of producing the antibody of the present invention is not limited, but can be produced by the method described below. That is, lymphocytes are collected from the peripheral blood of the patient who produces an autologous antibody to GPVI, and hybridomas of the lymphocytes activated by in vitro immunization with the mouse myeloma cells are prepared. After obtaining the antibody that is produced by the hybridoma prepared, by selection of the antibody which has a binding ability to GPVI and an activity that suppresses a platelet aggregation by the collagen, cells that produce the antibody can be obtained. By culturing the cells, the antibody of the present invention can be obtained.

The antibody of the present invention can be prepared as the recombinant human antibody using publicly known methods (a lot of methods are developed since Nature, 312: 643, 1984, and Nature, 321: 522, 1986 were published, respectively). Firstly, from the cells that produce the antibody of the present invention, e.g. the lymphocytes, preferably, the hybridoma which produces anti-GPVI monoclonal antibody, nucleic acid encoding VH or VL, e.g. cDNA may be obtained, and the base sequence and the amino acid sequence are determined. Then, by inserting the obtained cDNA encoding VH and VL into the expression vector for animal cells comprising a gene encoding human antibody CH and/or human antibody CL that has been prepared from the same or other human cell, respectively, human antibody-expressing vector may be constructed. By introducing the vector into the animal cell and expressing it, the antibody of the present invention can be manufactured. A method of preparing the gene to be introduced to the animal cell is not limited, and may be obtained from genomic DNA or cDNA derived from hybridoma, by PCR from mRNA of hybridoma, or by the chemical synthesis.

The vector, into which to the nucleic acid encoding VH or VL of the antibody of the present invention is incorporated, is not always limited, but a vector or a vector for high expression that generally is used for expression of gene encoding protein and adapted to expression of the antibody gene is preferred. Preferred example includes a vector containing EF promoter and/or CMV enhancer, specifically pEF-BOS or the vactor used in EXAMPLES. In addition, the vector that the nucleic acid encoding VH or VL is incorporated is usually prepared independently, and co-transfected into host cells. However, the nucleic acid may be incorporated into a single expression vector.

The host cell, in which the expression vector is introduced, is not always limited, and the cell that generally is used for expression of gene encoding protein and adapted to expression of the antibody gene is preferred. For example, bacteria (Escherichia coli, etc.), actinomyces, yeasts, insect cell (SF9, etc.), mammalian cell (COS-1, CHO, myeloma cell, etc.) are included.

As the constant region of the human antibody to use for preparing a recombinant human antibody, any human antibody constant region, for example, Cγ1 and Cγ4 for the human antibody heavy chain constant region, and Cκ for the human antibody light chain constant region can be used.

As the antibody that contains the CDR sequence of the human, an antibody that is obtained from the human antibody phage library and human antibody-producing transgenic animal is included in addition to the naturally occurring antibody in the human body. The human antibody phage library is a library, wherein the active fragment of the antibody such as Fab, single-stranded antibody, etc. is expressed on the surface of phage by inserting the antibody gene prepared from the human B cell into the gene of phage. From the library, using a binding activity to the substrate that the antibody is immobilized as an index, phage that expresses the active fragment of the antibody having the desired antigen-binding activity can be recovered. Moreover, the active fragment of the antibody can be converted into the human antibody molecule consisting of two intact H-chains and two intact L-chains by the genetically engineered techniques.

The present invention includes, in addition to the antibody consisting of two heavy chains and two light chains, the active-fragment of the antibody of the present invention. For example, the active fragment of the antibody includes Fab (fragment of antigen binding), Fab', F(ab')₂. The substance that the active fragment of the antibody is linked by linker and so on includes, for example, single-stranded antibody (single chain Fv: scFv) and disulfide stabilized Fv: dsFv). The peptide that contains the active fragment of the antibody includes, for example, a peptide containing CDR. These can be manufactured by the method of processing the antibody of the present invention with the suitable protease or the publicly known methods such as the recombinant DNA techniques.

Fab of the present invention can be obtained by treating the atrti-GPVI antibody of the present invention with pepsin, the proteolytic enzyme in case of IgM, or by processing it with the protease papain in case of IgG. Alternatively, Fab can be produced by inserting DNA encoding Fab of the antibody into prokaryotic or eukaryotic expression vector, introducing the vector into procaryotes or eukaryotes, and expressing the same.

F(ab')2 of the present invention can be obtained by treating the anti-GPVI antibody of the present invention with pepsin, the proteolytic enzyme. Alternatively, it can be prepared by linking the following Fab' with the thioether bond or the disulfide bond.

Fab' of the present invention can be obtained by treating F(ab')2 that specifically reacts to GPVI with the reducing agent, dithiothreitol.

As VH and VL that is contained in scFv of the present invention, those derived from either the antibody or the human antibody, which the hybridoma of the present invention produces, can be used. The scFv of the present invention can be manufactured by obtaining cDNA encoding VH and VL of the anti-GPVI antibody of the present invention, constructing the DNA encoding scFv, inserting the DNA into prokaryotic expression vector or eukaryotic expression vector, and introducing the vector into prokaryotes or eukaryotes to express the vector.

The term "dsFv" means an antibody that two polypeptides, wherein one amino acid residue in each of VH and VL is substituted with cysteine residue, bind each other via disulfide bond between said cysteine residues. The amino acid residue to be substituted for the cysteine residue can be selected based on the three-dimensional structure prediction of the antibody according to the method shown by Reiter et al. [Protein Engineering, 7, 697 (1994)]. As for VH and VL that is contained in dsFv of the present invention, the ones which are derived from the antibody of either the first or second embodiment of the present invention.

The dsFv of the present invention can be manufactured by obtaining cDNA encoding VH and VL of the anti-GPVI antibody of the present invention, constructing DNA encoding dsFv, inserting the DNA into prokaryotic or eukaryotic expression vector, and introducing the expression vector into prokaryotes or eukaryotes to express the same.

The peptide containing CDR is constituted by including at least one region or more of the H-chain CDR or of L-chain CDR. Plural CDRs can be bound directly or through the appropriate peptide linker. The peptide that contains CDR of the present invention can be manufactured by obtaining cDNA encoding VH and VL of the anti-GPVI antibody of the present invention, constructing DNA encoding CDR, inserting the DNA into prokaryotic or eukaryotic expression vector, and introducing the expression vector into prokaryotes or eukaryotes to express the same. Alternatively, the peptide that contains CDR can be manufactured by the chemical synthesis such as the Fmoc method (the fluorenylmethyloxycarbonyl method), the tBoc method (t-butyloxycarbonyl method), or the like.

The antibody of the present invention includes, for example, a human antibody that is produced by hybridoma, a human antibody that is produced by the cell transformed with EBV, a recombinant human antibody expressed from cDNA, or an antibody, wherein a radioisotope, protein, peptide or low molecular, etc. is conjugated with the active fragment of the antibody. To the N-terminal or the C-terminal end of H-chain or L-chain in the anti- GPVI antibody of the present invention or an active fragment thereof, an appropriate substituent or a side chain in the antibody or an active fragment of the antibody, and further a sugar chain in the antibody or an active fragment of the antibody, a radioisotope, protein, peptide or low molecular compound, etc. can be conjugated by a chemical method [The introduction to antibody engineering (Koutai kougaku nyuumon) (written by Osamu Kanemitsu, 1994, Chijin Shokan)]. Hybridoma means a cell that produces the monoclonal antibody having the desired antigen specificity, wherein it is obtained by fusing a lymphocyte with the myeloma cell derived from human, mouse, rat and so on.

When preparing a monoclonal antibody, in consideration of the compatibility with myeloma cell used for the cell fusion, selection is preferably performed. As for the myeloma cell, publicly known various cells are usable. These include SKO-007 from human, SHM-D33, which is a human-mouse heterozygous myeloma, P3, P3U1, SP2/O, NS-1 derived from mouse, and YB2/0 and Y3-Ag1 through Ag3 from rat.

The cell used for the preparation of hybridoma is not always limited, and for at least one kind among plural cells used for the preparation of hybridoma, preferred is a cell derived from human. As the cell derived from human, human lymphocyte in the peripheral blood, the lymph node or the spleen are used, and especially the human lymphocyte that the production of the autologous antibody is confirmed is preferable.

Activation of lymphocyte can be according to the publicly known method. For example, preferred are a method of preparing hybridoma with the myeloma cell derived from the human B cell or the mouse myeloma cell by collecting B cell from peripheral blood or spleen of the human and stimulating an antigen with in vitro immunization, a method of fusing with the mouse myeloma cell by transforming with EBV, and a method of fusing by stimulating with mitogen such as PWM and activating B cell to polyclonal antibody (Immunological experiment procedures (Men-eki jikken sousa-hou) I and II, edited by Shunsuke Migita et al., Nankoudo).

The antigen used for stimulation of the cell is not always limited. The animal, from which the protein as an antigen is originated, can be appropriately selected for any purpose of the antibody. The protein as an antigen may be naturally occurring product, genetically engineered product, chemically synthesized product, or fusion protein with other protein or peptide, and the like. For example, the platelet, the membrane of the platelet, purified GPVI, recombinant GPVI, and GPVI-Fc, preferably GPVI-Fc can be used.

Fusion of the activated lymphocytes with myeloma cells can be performed using the publicly known methods such as the method by Milstein et al. (Methods in Enzymol., volume 73, pages 3). The methods include, for example, the method using polyethylene glycol (PEG) as a fusing agent (Introduction to the monoclonal antilbody experiment procedure (Tan-kuron koutai jikken sousahou nyuumon), written by Tamie Ando and Takeshi Chiba, Kodansha) or the electrofusion method. The mixing ratio of the immunocyte and the myeloma cell is not limited as long as it is the ratio that the cells can be fused. Preferably, 1/10 to equal amount of the myeloma cells to the activated lymphocytes may be used. In the cell fusion using PEG (average molecular weight: 1,000-4,000), PEG concentration is not always limited, but 50% is preferable. In addition, as the fusion efficiency accelerator, auxiliary substance such as dimethylsulfoxide (DMSO) may be added. The fusion is started by adding pre-warmed PEG solution at 37°C to the mixed cells, and is terminated by adding medium after the reaction for 1-5 minutes.

The hybridoma, which was formed by this fusion is cultured for one (1) to ten (10) days in selection medium such as the medium containing hypoxanthine, thymidine and aminopterin (HAT medium) to isolate unfused cells. The obtained hybridoma is further selected based on the antibody to be produced. The selected hybridoma is isolated to a single clone by the publicly known limiting dilution. Thereby, a monoclonal antibody-producing hybridoma is established.

For a method of detecting the activity of the antibody that is produced by the hybridoma, the publicly known method can be used. Herein, the activity of the antibody is detected in the following two steps: the binding ability to GPVI antigen as the first step and the activity of inhibiting the binding of GPVI and collagen as the second step. Examples of the detection method for the first step include ELISA, Western blotting, radioimmunoassay and the like. As the detection method for the second step, ELISA (inhibiting the binding), protein interaction analysis (BIACORE and so on), a platelet aggregation suppression assay are given.

The established hybridoma can be cultivated by the publicly known method, and from its culture supernatant a monoclonal antibody can be obtained.

The antibody can be purified using the publicly known purification means such as the salting-out method, gel filtration, ion exchange chromatography or affinity chromatography.

The concentration of the antibody can be measured by the publicly known quantification method of protein, e.g. the measurement of absorbance at 280 nm absorbance.

For a method of confirming the antigen binding property of the anti-GPVI antibody of the present invention or a method of detecting GPVI in the biological sample using the anti-GPVI antibody of the present invention, fluorescence antibody technique, enzyme linked immunosorbent assay (ELISA), radioimmunoassay (RIA), immunohistochemical method such as immunohistological staining and immunocytological staining (ABC method, the CSA method and so on), the Western blotting method, immuno-precipitation method, enzyme-linked immunoassay as described above, sandwich ELISA method [Tan-kuron koutai jikken manual (The Monoclonal Antilbody Experiment Manual) (Kodansha Scientific, 1987), Zoku seikagaku jikken kouza (The Continued Biochemical Experiment Course 5) Menneki-seikagaku kenkyuuhou (Immunobiochemical Research Method) (TOKYO KAGAKU DOZIN, 1986)] can be used.

A method of assaying an effect of the antibody of the present invention on human platelet aggregation by the substances that induces a platelet aggregation like collagen is not always limited, and can be done by the conventional method. Specifically, the antibody of the present invention is added to human platelet suspension, and then collagen is added. The aggregation rate is measured with the platelet aggregation activity analyzer.

A method of measuring a human platelet aggregation by only the antibody is not always limited and can be done by the conventional method. Specifically, the antibody of the present invention is added to human platelet suspension, and an aggregation rate is measured with the platelet aggregation activity analyzer and so on.

### (Uses)

The antibody of the present invention is an antibody that specifically binds to human GPVI. The antibody of the present invention, the active fragment of the antibody, the modified antibody that binds to the chemicals or the composition that comprises these mixtures have a variety of uses including prevention, diagnosis and treatment of the human diseases, and detection of human GPVI in test sample, cells, tissues and the like.

Especially in one embodiment of the antibody of the present invention, since the antibody cannot occur a human platelet aggregation solely, the antibody itself in addition to the active fragment of the antibody can suppress the human platelet aggregation. Therefore the antibody itself can be used for prevention, diagnosis and treatment of the human disease by administering to the human, without preparing an active fragment.

### Uses: Medicaments

Since the antibody of the present invention has a high specificity for binding to GPVI, and is derived from the human, and preferably solely has no activity that enhances or induces a human platelet aggregation, in particular, it is useful for prevention and/or treatment of human diseases, for example, diseases caused by activation or aggregation of platelet, or vascular endothelial disorders or arteriosclerotic reaction. In addition, it can be used for prevention and/or treatment of diseases caused by blood clot or embolus such as thrombosis, embolism and the like. Examples of these diseases include venous thrombosis as well as arterial thrombosis, or brain infarction caused by atrial fbrillation.

Specific examples of human diseases or clinical conditions that are able to prevent and treat by the antibody of the present invention include vascular endothelial tylosis, re-angiostenosis, angina pectoris or heart infarction at the time of heart infarction, thrombolytic therapy, percutaneous transsluminal coronary angioplasty (PTCA), stent implementation, bypass surgery or synthetic fgraft, or thereafter; atrial fibrillation or atrial flutter, and thrombosis caused by these diseases; embolism or brain infarction; thromboangiitis obliterans; acute arterial occlusion; arteriosclerosis obliterans or deep venous thrombosis; brain infarction (atheromatous thrombotic infarction, lacunar infarction, cardiogenic infarction); transient ischemic attack; cerebrovascular spasm after subarachnoid bleeding; pulmonary blood clot; pulmonary embolism; vascular purpura; idiopathic thrombocytopenic purpura; thrombotic thrombocytopenic purpura; disseminated intravascular coagulation; prevention of blood coagulation at the time of extracorporeal circulation; systemic lupus erythematosus; multiple arteritis; antiphospholipid antibody syndrome; purpura nephritis; endothelial cell injury associated with diabetes mellitis; diabetic nephritis; diabetic retinopathy; nephritic embolism; complications associated with transplantation (veno-occlusive disease of the liver, graft-versus-host disease); and so on.

The antibody of the present invention can be administered to the diseases to be objective for the aforementioned prevention and/ treatment solely or in combination with other pharmacologically active ingredient. Examples of such pharmacologically active ingredient include publicly known thrombolytic agent such as tissue plasminogen activator(t-PA) and derivatives thereof (including variant or so-called second generation); urokinase, streptokinase or publicly known platelet inhibitor (e.g. aspirin, ticlopidine, clopidogrel, thromboxane antagonist, thromboxane synthesis inhibitor, GPIIb/GPIIIa antagonist); publicly known anticoagulant (e.g. warfarin, heparin, low molecular heparin, pentasaccharide, thrombin inhibitor, FXa inhibitor, FVIIa inhibitor); and the like. Herein, the term "combination" includes the case where combination of drugs comprising both the antibody of the present invention and the pharmacologically active ingredient is administered and the case where the antibody of the present invention and the pharmacologically active ingredient are administered as an independent formulation at the same time or time difference, and for the dosage form, no object as long as both exist simultaneously in the blood.

A medicament comprising the antibody of the present invention and pharmaceutically acceptable somposition as an effective ingredient can be prepared as tablet, injectable solution, powdered drug, suppository and the like using carrier for formulation, excipient and other additives used generally, and administered to human and other animals.

When applied to the human, route for administration includes oral, intraveneous (bolus, continuous drip, intermittent drip), subcutaneous, intramuscular, intra-articular, transdermal, and transnasal administration. In general, oral administration or intraveneous administration may be used. Clinical dose of the antibody of the present invention to the human may appropriately be determined in consideration of conditions, body weight, ages, sex, etc. of the patient to be administered. In general, to adult, by intraveneous administration, dose of 1-10000 mg, preferably 10-1000 mg per day may be used, and the amount can be administered at one time or within several times. Since dosage varies depending on various conditions, there is a case where the amount less than the above-mentioned range is effectively administered.

Herein, the antibody of the present invention includes various antibodies having different mechanisms meanwhile sharing recognition of GPVI in common. For example, since in the antibody that directly inhibits the binding of GPVI to collagen, or that suppresses activation and/or aggregation of platelet by cleaving GPVI, a relatively immediate effect can be expected, there have possibilities that the antibody is useful at least in the acute period of the disease (for example, in the time of myocardial infarction or PTCA implementation, or right before or after the events). In such a case, preferably, to make the antibody of the present invention bind to most of GPVI on the surface of platelet in the blood, relatively massive antibodies can be administered, e.g. by a single or divided intravenous injection or an intravenous drip. Also, in the antibody, in which GPVI is incorporated internally, a continuous effect, but not an immediate effect can be expected considering a life-time of human platelet in the blood (about 9-10 days) and a half-life period of human antibody in the blood (in the case of IgG, several weeks). Thus, for example, there have a possibility that it is useful in chronic stage of diseases (several day to several month after development of myocardial infarction or PTCA implementation). In such a case, the antibody, whose amount is necessitated for making GPVI on the platelet surface disappear to the extent of inhibiting a reactivity to platelet collagen in the blood partially, preferably fully, can be administered at intervals of relatively long duration, e.g. from several days to several weeks per cycle, for example, by single dose or divisional intravenous injection, or the intravenous drip. Therefore, in the preferred embodiment, the antibody of the present invention may possess these effects in parallel. In addition, a treatment, wherein multiple anti-GPVI antibodies that respective effects can be expected is combined, may be performed.

A composition for parenteral administration generally includes a solution of the immunoglobulin dissolved into the acceptable carrier, preferably aqueous carrier or its mixture. Various aqueous carriers such as water, buffer solution, a phosphate buffered saline (PBS), 0.4% of physiological saline, 0.3% of glycine, human albumin solution and the like can be used. These solutions are aseptic, and generally, microparticle material does not exist in these solutions. These compositions can be sterilized by the conventional and well-known method of sterilization. To approximate physiological conditions, the compositions may be included, on demand, pharmacologically acceptable auxiliary substance such as pH adjusting and buffering agent, toxicity regulating agent, and the like, specifically sodium acetate, sodium chloride, potassium chloride, calcium chloride and the sodium. The concentration of the antibody in these formulations can vary extensively, i.e. change from less than about 0.005% by weight (usually, at least about 1& by weight) to the large quantity, i.e. 15% or 20% by weight, and may be selected according to the selected and specific mode of administration, mainly based on the volume of the solution, the viscosity and the like.

An actual method of preparing a parenteral administration composition is publicly known or obvious for persons skilled in the art, and further is described in detail in Remington's Pharmaceutical Sciences (15th Edition, Mack Publishing Company, Easton, Pennsylvania, 1980), wherein the reference is incorporated by reference in its entirety. A composition suitable for wash (lavage) or the other route is selected according to the intended specific use. Some pharmaceutical compositions can include an anti-GPVI antibody and the other treatment agent which is regularly used in the disease. In any cases, the bolus administration and the continuous administration can be applied. In addition, an effective amount for prevention or treatment is arbitrarily determined depending on object diseases, clinical condition and the condition of the patient and the like.

The antibody of the present invention is frozen or lyophilized for storage, and prior to use, can be reconstituted in the suitable carrier. This technique is known to be effective in the conventional immunoglobulin. Further the publicly known techniques for lyophilization and reconstitution can be used. It is recognized for persons skilled in the art that lyophilization and reconstitution bring about activity loss of the antibody at various degrees (e.g. in the conventional immunoglobulin, IgM antibody, larger activity loss than that of IgG antibody tends to occur), and that use level may have to be regulated for compensation of loss.

### Use: Detection of GPVI

A method of detecting GPVI in test sample using the antibody of the present invention or an active fragment thereof comprises the process, in which the test sample is brought in contact with the antibody of the present invention or an active fragment thereof, and the process, in which GPVI in the test sample bound to the antibody of the present invention or an active fragment thereof. The process for quantifying GPVI in the test sample may further be included. Using the method of detecting GPVI in test sample, diseases can be diagnosed. In particular, it is possible to use for diagnosing human diseases such as thrombotic, embolic or arteriosclerosis diseases.

Examples of the method of detecting GPVI in the test sample using the antibodyof the present invention includes, but is limited to, the sandwich ELISA system, the inhibition-ELISA system, fluorescence antibody method, immunohistochemical staining method, radioisotope-labeling immune antibody method, Western blotting method, immunoprecipitation method and so on. As a target test sample, a biological sample is used, but is not limited. Examples of the sample include body fluid, tissues or cells from animal, particularly human, bacterial cells, and extracts thereof, culture supernatant, smears and sections, but preferred is platelet.

A method of inhibiting GPVI or a binding of GPVI on the platelet to collagen using the antibody of the present invention or an active fragment thereof comprises at least one process among the process of contacting GPVI or GPVI on the surface of platelet with the antibody of the present invention or an active fragment thereof, and the process of inhibiting GPVI or a binding of GPVI on the platelet to collagen by the antibody of the present invention or an active fragment thereof.

With respect to the process of detecting a inhibition for platelet aggregation of GPVI or GPVI on the platelet by the antibody of the present invention or an active fragment thereof, it is possible to use in vivo assay system as well as the aforementioned in vitro assay system. The term "in vivo assay system" means an evaluation system that detects an influence for vital functions or status given by the antibody of the present invention or an active fragment thereof with administration of the antibody or an active fragment thereof to the living body. For example, a system that evaluates an influence given to an index for severity of disease by administering the antibody of the present invention or an active fragment thereof to collagen-injected model animal is exemplified.

### EXAMPLES

The present invention will be further described in detail with reference to the following EXAMPLES, but these examples are given by way of illustration. Further, the present invention should not be limited by these EXAMPLES. In addition, codes used in the following description are based on the conventional designation in the art.

### EXAMPLE 1 Preparation of a human soluble GPVI-Fc

In order to use as the antigen for in vitro immunization and for screening, a fusion protein consisting of the extracellular domain of human GPVI and Fc fragment of human IgG (GPVI-Fc). In addition, DNA manipulation was according to Molecular Cloning, A Laboratory Manual 3rd ed., Joseph S., et al., Cold Spring Harbor Laboratory Press (2001) unless otherwise noted.

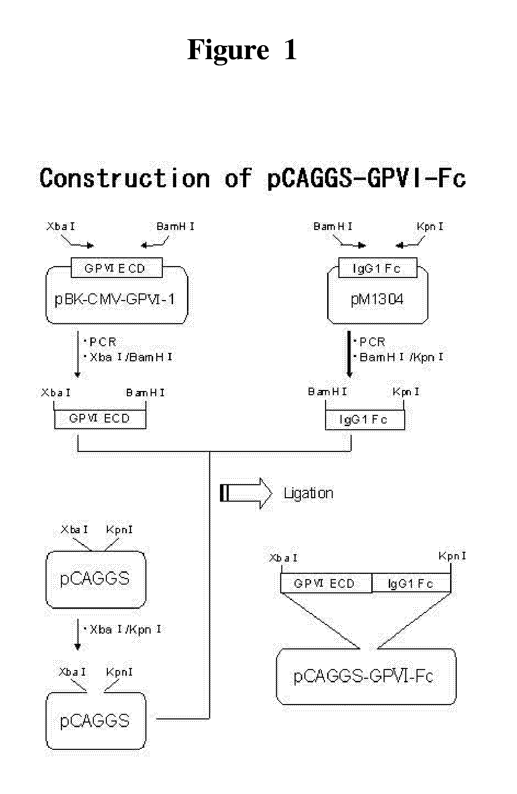

GPVI-Fc expressing plasmid was prepared by genetic engineering using the following procedure. Using a plasmid pBK-CMV-GPVI-1, in which human GPVI cDNA cloned by Esumi et al. is integrated (Biochem. Biophys. Res. Commun. 2000 Oct 14; 277(1): 27-36), as a template, sense primer 1 (SEQ ID NO: 33; including a restriction enzyme XbaI recognition sequence at the. 5' end) and antisense primer 1 (SEQ ID NO: 34; including a restriction enzyme BamH I recognition sequence on the side of the. 5' end), PCR was performed to get the cDNA encoding a human GPVI extracellular domain (269 amino acids). On the other hand, using the plasmid pM1304 (described in WO97/42319), in which the human IgG₁Fc domain cDNA is contained, as a template, PCR was done to get the cDNA encoding the human IgG₁Fc domain (506 amino acids) connectable with the GPVI extracellular domain in frame. Sense primer 2 for the PCR (SEQ ID NO: 35) contained the cDNA sequence encoding the N-terminal region of the human IgG₁Fc domain, and further was designed to allocate the restriction enzyme Bam HI recognition sequence at the 5' end. Also, as antisense primer 2 (SEQ ID NO: 36), the sequence designed to allocate the restriction enzyme KpnI recognition sequence at the C-terminal side of the human IgG₁Fc domain was used. After treating two cDNA fragments obtained by PCR with restriction enzymes, the fragments were inserted into the cloning site of pCAGGS (Patent Gazette No. 2824434), the expression plasmid whose host is a mammalian cell. That is, the cDNA fragment encoding the human GPVI extracellular domain was cut with the restriction enzymes Xba I and Bam HI, and the cDNA fragment encoding the human IgG₁Fc domain was cut with the restriction enzymes Bam HI and Kpn I. A linker was inserted into the cloning site of pCAGGS to add suitable restriction enzyme cleavage sites (Xba I and Kpn I). Subsequently, the cDNA encoding the human GPVI extracellular domain and the cDNA encoding the human IgG₁Fc domain were ligated in frame and inserted into the cloning site. This process is shown in Figure 1.

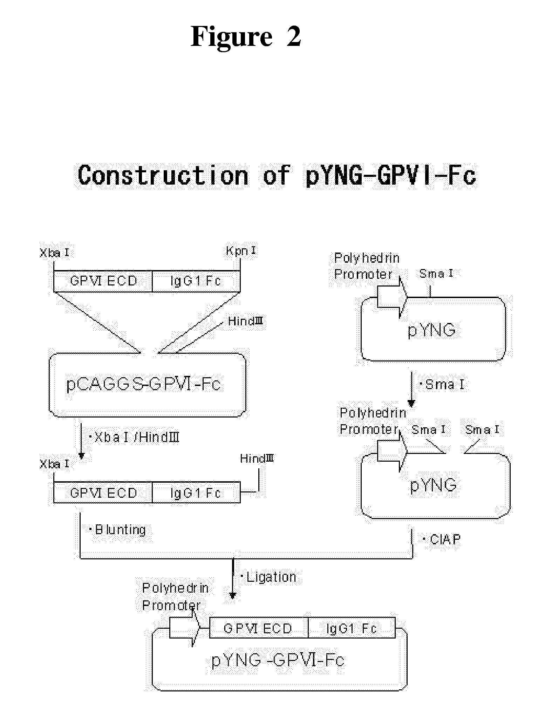

Based on the obtained expression plasmid (pCAGGS-GPVI-Fc), a transfer vector to Baculovirus and a recombinant virus were prepared, and expression with the silkworm pupa was performed. That is, the DNA fragment encoding GPVI-Fc was excised by cutting pCAGGS-GPVI-Fc with the restriction enzymes Xba I and in Hind III, and its end was blunted using the Blunting Kit (TAKARA). The DNA fragment was inserted into the Sma I site of pYNG, the transfer vector to Baculovirus, and the clone, in which the fragment is inserted in the forward direction the polyhedrin promoter (pYNG-GPVI-Fc) was selected. This process is shown in Figure 2. Based on the clone, a recombinant virus was prepared, and an expression of the GPVI-Fc protein in the virus-infected cells was confirmed by Western blotting. By infecting the virus solution which was finally prepared to a silkworm pupa, the expression of GPVI-Fc was performed.

As a result, the expression of GPVI-Fc with enough quantity was detected in the extract of the silkworm pupa. From the extract, GPVI-Fc was purified by applying to the Protein A column (Prosep-A, Milipore).

### EXAMPLE 2 Preparation of anti-GPVI monoclonal human antibody using human peripheral blood lymphocytes having an anti-GPVI autologous antibody

Anti-GPVI monoclonal human antibody was prepared as follows by fusing lymphocytes from a donor who is confirmed to have an autologous antibody to GPVI with myeloma cells. Firstly, by layering 6 ml of heparin added blood aseptically collected from the donor who gave informed consent in writing to Leucosep (Greiner) which added 3 ml of Ficoll-Plus (Amersham Pharmacia Biotech AB) and centrifuging at 1000g, lymphocyte fraction was recovered. After twice washing the obtained lymphocyte fraction with Dulbecco-PBS (hereinafter, sometimes referred to as D-PBS) by centrifugation at 800g, the fraction was suspended in Hybridoma-SFM (Invitrogen) containing 10% FCS (fetal bovine serum) to get 7.4x10⁷ cells.

To the above-mentioned lymphocyte fraction, PHA-L (Sigma), LPS (DIFCO) and the purified GPVI-Fc described in EXAMPLE 1 were added to be 2.5 µg/ml, 20 µg/ml and 10 µg/ml, respectively. By adjusting the cell density to 1x10⁶ cells/ml and culturing for three days, a lymphocyte was activated. Moreover, at the time of cultivation, by further addition of 400 U/mL IL-4 (PeproTech) and ad libitum fine-tuning to the conditions, for example, prolonging an incubation period to eight days, an activation of lymphocytes was tested. According to Ando et al. ("Tan-kuro-n Koutai Jikken Sousa-hou Nyumon" ("Introduction to Experimental Methods for the Monoclonal Antilbody") written by Tamie Ando and Takesi Chiba, Kodansha), the activated human lymphocytes and the mouse myeloma cells (SP2/O-Ag14, ATCC CRL1581) were mixed at the ratio of 4:1, and the cells were fused with 50% polyethylene glycol (Sigma). After completion of the fusion, the fused cells were suspended in Hybridoma-SFM containing 10% FCS, 1 × HAT (Invitrogen) and cultured for ten days. After cultivation, the culture supernatant of the grown hybridoma was harvested, and the hybridoma which was producing a desired antibody by ELISA method.

That is, to the immunoplate (Maxisorb, NUNC), on which 0.25 µg/well of the purified GPVI-Fc were immobilized, 50 µL of the culture supernatant of the hybridoma was added, and the reaction at 37°C for one hour was performed. As a control, to the well, on which the human purified Fc (Athens Research And Technology, Inc.) was immobilized in a similar way, the culture supernatant was added to react. After completion of the reaction, each well was washed, and peroxidase-labeled anti-human kappa antibody (DAKO, P129) or peroxidase-labeled anti-human lambda antibody (DAKO, P130) was added to the well. The reaction was performed at 37°C for one hour, and the well was washed in a similar way. TMB coloring solution (BioFix) was added to each well to react for ten minutes. Then 0.5 M sulfuric acid solution was added to terminate the reaction. Further, by measuring an absorbance at 450 nm with spectrophotometer for plate, the well, in which the absorbance in the case of the purified human Fc did not increase, meanwhile that only in the case of the GPVI-Fc immobilized well increased, that is, the well, in which the anti-GPVI antibody-producing hybridoma is present, was selected.

Moreover, culture by limiting dilution was performed to obtain a single clone. That is, for the culture fluid after ten days cultivation, a screening was done as described above to get a hybridoma that produces the anti-GPVI monoclonal human antibody.

The selected hybridoma was cultured in the Hybridoma-SFM containing 10% FCS, and the medium was replaced with the serum-free medium to produce the antibody. IgM antibody was purified by Prosep-ThiosorbM column (MILLIPORE) according to the manual, and IgG antibodywas purified using Prosep-A column (MILLIPORE). The purified antibody was dialyzed against 0.076 M phosphate buffer (PBS) (pH6.4), and the concentration was calculated from the absorbance at 280 nm.

### EXAMPLE 3 Typing of the anti-GPVI monoclonal human antibody prepared

Typing of the antibody obtained in EXAMPLE 2 was done with the Human IgG Subclass Profile ELISA Kit (Zymed Laboratories) and the Western blotting. The ELISA was performed according to the manual, and the diluents of the antibody as the sample were used. For the antibody that cannot be detected with the kit, about one microgram of each sample was isolated on 4-20% SDS-PAGE and transfererred onto PVDF membrane (MILLIPORE) to analyze by Western blotting. That is, after blocking the PVDF membrane, peroxidase-labeled rabbit anti-human IgM antibody (P0322, DAKO) was reacted. After washing, by reacting with ECL reagent (Amersham Pharmacia Biotech AB), the band which reacts with light capture (ATTO) was detected. The results of typing of the obtained anti-GPVI antibody were shown in Table 1.

**Table 1 Results of typing**

| (1) When cultivating for 3 days in the presence of PHA-L and the LPS | |
|---|---|
| Clone number | Sub-class |
| #2-4 | IgM/λ |
| #2-6 | IgM/λ |
| #2-7 | IgM/λ |
| #2-16 | IgM/κ |
| #2-37 | IgM/λ |

| (2) When cultivating for 8 days in the presence of PHA-L, LPS and the IL-4 | |
|---|---|
| Clone number | Sub-class |
| #4-1 | IgG2/λ |
| #4-7 | IgG2/λ |
| #4-28 | IgM/κ |
| #4-43 | IgM/λ |
| #4-44 | IgM/λ |
| #4-45 | IgM/λ |
| #4-52 | IgM/λ |
| #4-56 | IgM/κ |
| #4-62 | IgM/λ |
| #4-65 | IgM/λ |
| #4-68 | IgM/κ |

### EXAMPLE 4 Assay for the GPVI binding activity of the anti-GPVI monoclonal human antibody (ELISA method)

GPVI binding activity of the purified antibody that was obtained in EXAMPLE 2 was assayed by the ELISA method that was described in EXAMPLE 2. As substitute for the culture supernatant of hybridoma in EXAMPLE 2, 20 ng of the purified anti-GPVI monoclonal human antibody prepared in EXAMPLE 2 was added, and as a control, purified human IgM (Cappel) was used.

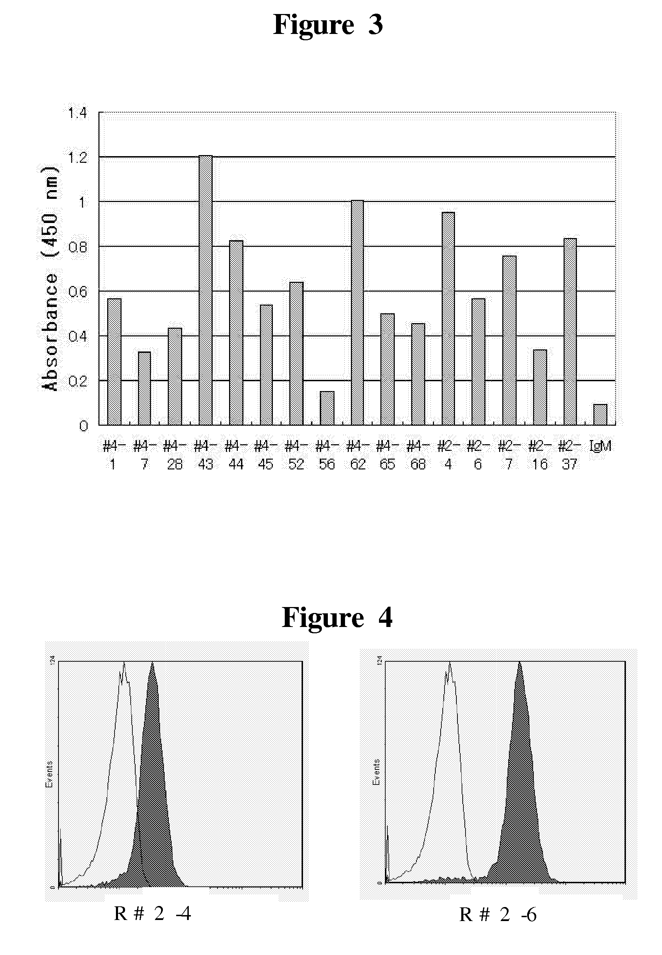

As a result, as shown in Figure 3, in the case of the human IgM that were used as a control, the absorbance did not increase, whereas in either cases of the purified anti-GPVI monoclonal human antibodies that are produced from the selected hybridomas, remarkable increasing of the absorbance rises was admitted. Thus it was confirmed that the prepared antibody specifically recognizes GPVI.

### EXAMPLE 5 Study on inhibition for GPVI-Fc binding to collagen by the anti-GPVI monoclonal human antibody

For the purpose of determining if the antibody obtained in EXAMPLE 2 specifically inhibits the binding between GPVI and collagen, the analysis using the protein interaction analyzer (BIACORE3000) was performed. Firstly, according to the manual by BIACORE Inc., a human collagen Type I (Seikagaku Kogyo) of 6303 RU (Resonance Unit) was fixed on CM5 chip (BIACORE). Herein, the term "RU" is a unit that represents response used in BIACORE equipment, and 1000 RU shows a result, to which 1.2 ng of substance is bound. After admixing GPVI-Fc with purified human IgM or purified anti-GPVI antibody to make the concentration 50 µg/ml each, the mixture was injected to the collagen-fixed chip.

### EXAMPLE 6 Influence of the antibody on human platelet agglutinability

Using the blood collected from norma healthy subject whose consent was obtained in writing, according to conventional manner (Takayama H et al., Biochemical and Biophysical Research Communications, 174, pp. 922-927 (1991)), platelet was prepared and used at the final concentration of 2 x 10⁸ through 3 x 10⁸ cells/mL. Assay for platelet agglutinability was performed as follows according to Ezumi Y et al. (Blood, 99, pp. 3250-3255 (2002)). After adding the antibody obtained in EXAMPLE 2 or the solvent that are a test sample, it was incubated at 37°C for 5 minutes. Further, CaCl2 solution was added to make the final concentration 1 mM. Incubation at 37°C for 3 minutes with stirring was performed. To the mixture, the solution of collagen (NYCOMED PHARMA GMBH) was added to make the final concentration 3 to 4 µg/mL. By measuring a light transmission with the passage of time using the platelet-agglutinability analyzer (Kowa Co., Ltd. Inc. PA-200), a function for suppressing a collagen-induced platelet aggregation of the antibody was assayed. The result was shown in Table 2. In addition, the result of measurement of the platelet aggregation was represented by transmittance (the maximum-aggregation-rate) when the light transmission becomes maximum.

Next, by adding a human thrombin (Sigma) as a platelet-inducing substance instead of collagen to make the concentration 0.3 units/ml, influence on a platelet aggregation was measured in a similar manner. As a result, the maximum-aggregation-rate at the antibody concentration of 10 µg/mL was 96% in case of the #2-4 antibody.

Moreover, in the aforementioned assay, when a platelet aggregation inducing function with the test sample (the antibody) only was measured without adding the platelet aggregation inducing material such as collagen and so on, the maximum-aggregation-rate at the antibody concentration of 10 µg/mL was 3% in case of the #2-4 antibody.

As described above, it was observed that the #2-4 antibody does not have a platelet aggregation inducing function solely, and suppress only a collagen-mediated platelet aggregation.

**Table 2 (A) The #2-4antibody**

| Final concentration of the antibody | Maximum aggregation rate (Collagen 4 µg/mL) |
|---|---|
| Control | 77% |
| 0.1 µg/mL | 63% |
| 0.3 µg/mL | 49% |
| 1 µg/mL | 27% |

### EXAMPLE 7 Determination of dissociation constant of the anti-GPVI monoclonal human antibody

A dissociation constant of the antibody, wherein a suppressing activity for platelet aggregation was observed in EXAMPLE 6, was determined using protein interaction analyzer (BIACORE3000). The purified GPVI-Fc described in EXAMPLE 1 was immobilized on the CM5 chip according to the manual of the BIACORE Inc.. The dissociation constant (Kd) of the #2-4 antibody was calculated to be 4.13 x 10⁻⁸ M when measuring in Wizard Program of BIACORE3000 with regard to the #2-4 antibody, and analyzing with the BIAevaluation software of the BIACORE Inc..

### EXAMPLE 8 Determination of amino acid sequence of CDR of the anti-GPVI antibody

The hybridoma selected by the screening using the ELISA method of EXAMPLE 2 was cultured according to EXAMPLE 2. At the stage of the cell density to be 2 x 10⁵ cells/ml, the culture fluid was harvested. From the obtained cells, using TRIzol (Invitrogen) mRNA was extracted. Next, according to the manual of Superscript First-strand synthesis System II (Invitrogen), a single-stranded cDNA was synthesized from the mRNA using the oligo dT primer. With reference to the information of the article (J. Immunol. Methods 1995 Feb 27; 179(2): 203-14 and J. Mol. Biol. 1991 Dec 5; 222(3): 581-97), PCR primers (the sequences are listed in Table 3) to amplify a heavy chain and light chain variable region were synthesized, and using the single-stranded cDNA from hybridoma previously prepared as a template PCR was performed. After detecting the amplified DNA band with 2% agarose, the PCR product was purified using spin column (Sigma). The purified PCR product was admixed with pT7BlueT vector (Novagen) to perform a ligation reaction at 16°C for 30 minutes using Ligation kit ver II (TAKARA). Using the reaction mixture, competent cell E. coli (JM109, TAKARA) was transformed, and the cells were plated on the LB plate containing X-Gal and IPTG and cultivated for overnight. White colonies appeared were picked up to confirm the insert into the vector by colony direct PCR using Ex Taq polymerase (TAKARA), U-19mer primer (the sequence is listed in Table 3), T7 promoter primer (the sequence is listed in Table 3, Novagen). Next, the colonies, in which the insert was confirmed, were cultivated with the LB medium for overnight, and the plasmids were purified using QIAGEN plasmid mini kit (QIAGEN). The purified plasmids were reacted with DYEnamic ET terminator cycle sequencing kit (Amersham Bioscience) using U-19mer primer and T7 promoter primer, and the sequences were analyzed using sequencer ABI PRISM3100 (Applied Biosystems).

CDR sequences determined were shown in Table 4. In addition, in the base sequence encoding VL CDR3 of the clone #2-4 (SEQ ID NO: 28), guanine at the 31 st position is not actually detected, but it is expected that it is a guanine from the result of the other clones. Based on the expectation, the amino acid sequences of VL CDR3 (SEQ ID NO: 12) and VL CDR (SEQ ID NO: 16) of the #2-4 clones are listed.

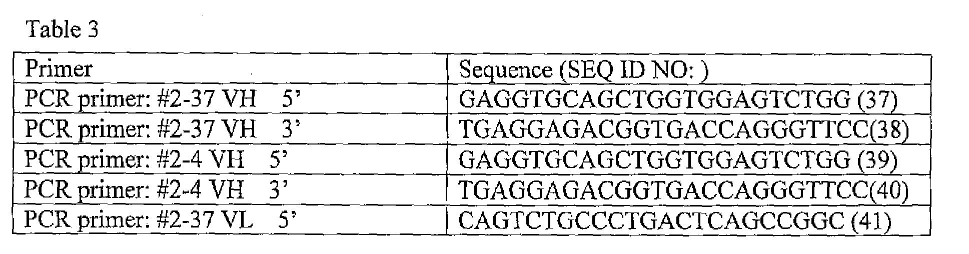

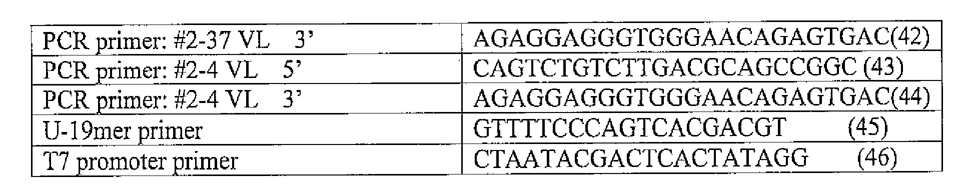

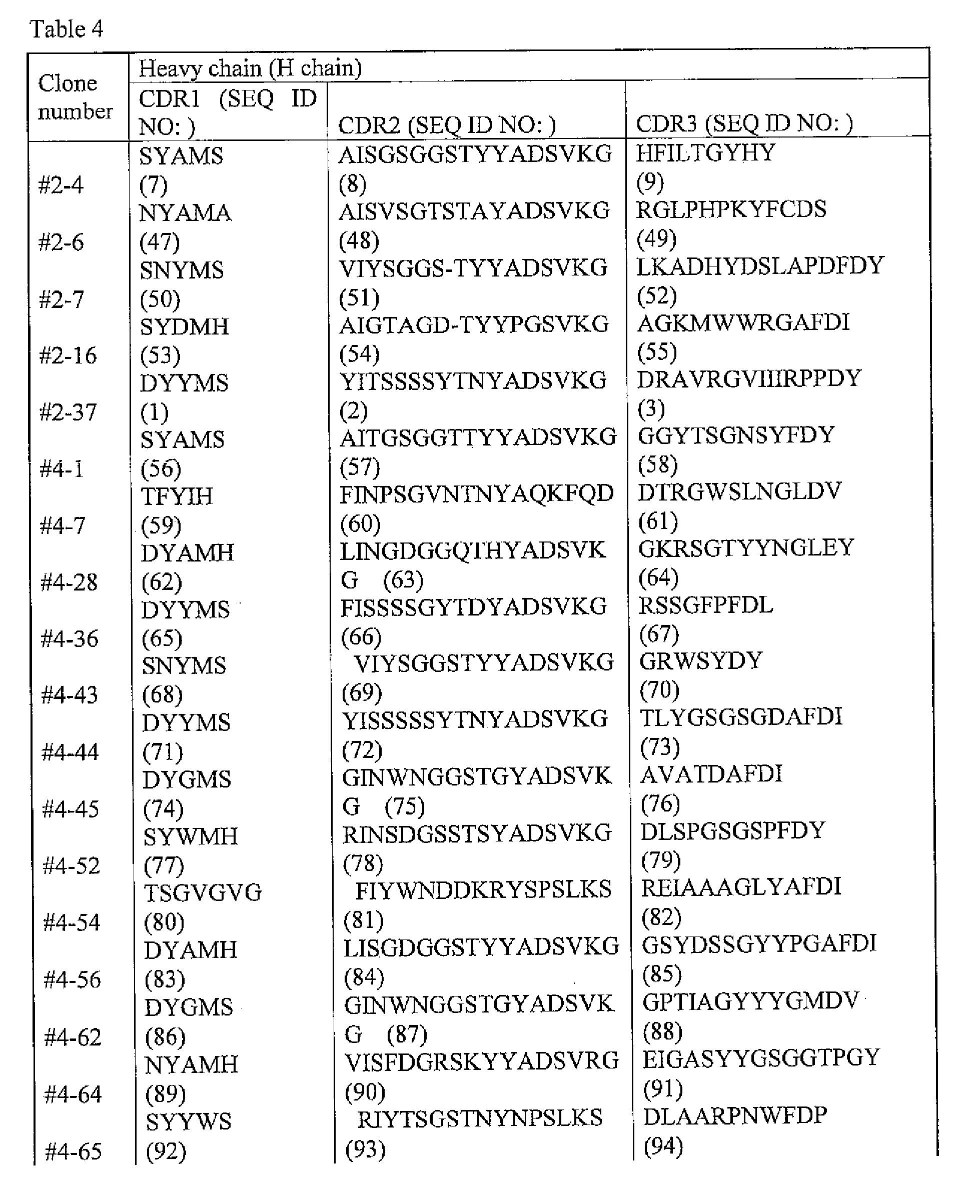

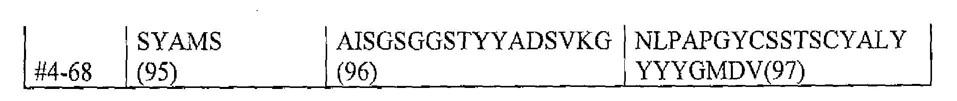

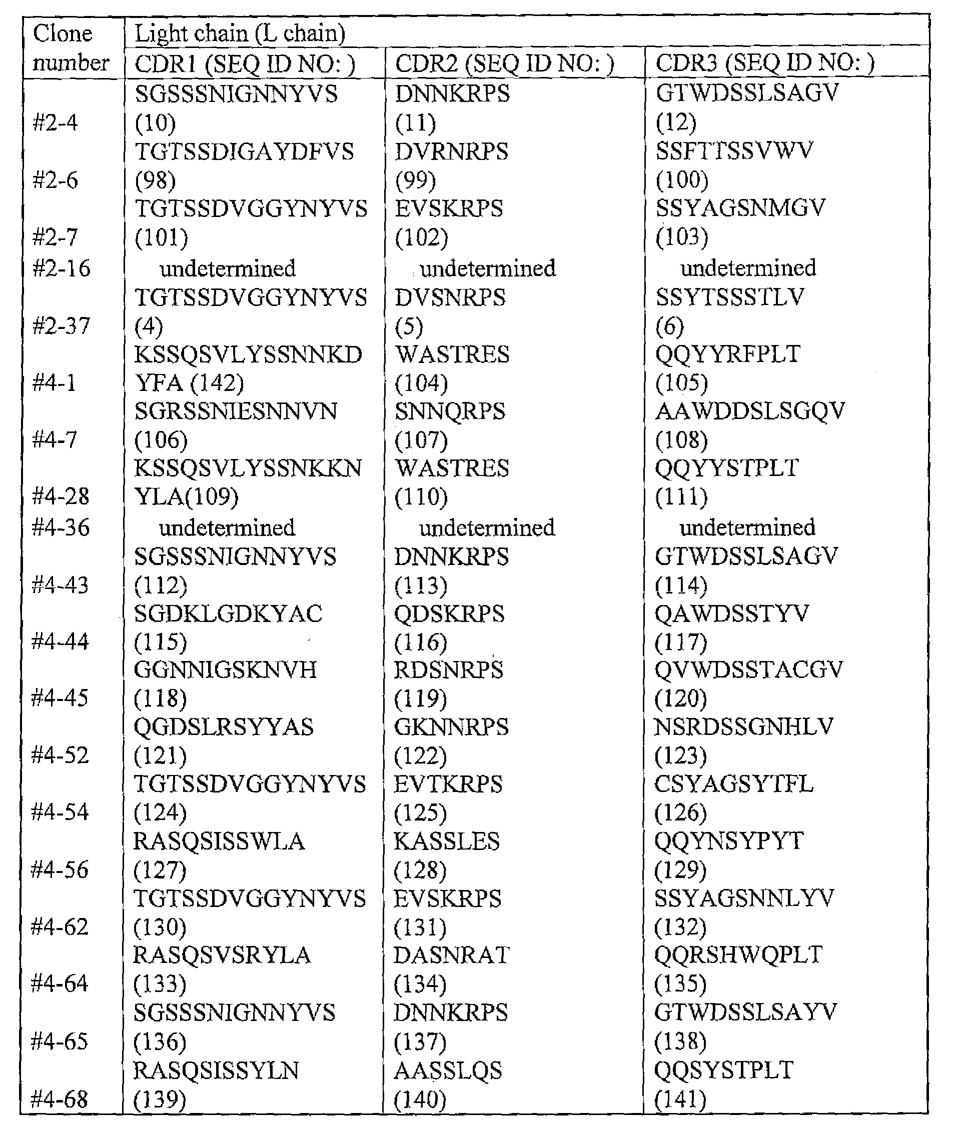

In addition, the amino acid sequence and the nucleotide sequence of the H chain and light chain variable regions of the #2-4 and #2-6 clones are shown in Table 5 and Table 6 respectively, and the nucleotide sequence of CDR of the #2-4 and #2-6 clones is shown in Table 7.

**Table 5**

| Clone number | H chain variable region amino acid sequence | Light chain variable region amino acid sequence |
|---|---|---|
| #2-4 | SEQ ID NO: 15 | SEQ ID NO: 16 |
| #2-6 | SEQ ID NO: 143 | SEQ ID NO: 144 |

**Table 6**

| Clone number | H chain variable region nucleotide sequence | Light chain variable region nucleotide sequence |
|---|---|---|
| #2-4 | SEQ ID NO: 31 | SEQ ID NO: 32 |
| #2-6 | SEQ ID NO: 145 | SEQ ID NO: 146 |

**Table 7**

| Clone number | Heavy chain (H-chain) | | |
|---|---|---|---|
| | CDR1 | CDR2 | CDR3 |
| #2-4 | SEQ ID NO: 23 | SEQ ID NO:24 | SEQ ID NO:25 |
| #2-6 | SEQ ID NO: 147 | SEQ ID NO: 148 | SEQ ID NO: 149 |

| Clone number | Light-chain (Light chain) | | |
|---|---|---|---|
| | CDR1 | CDR2 | CDR3 |
| #2-4 | SEQ ID NO: 26 | SEQ ID NO: 27 | SEQ ID NO: 28 |
| #2-6 | SEQ ID NO: 150 | SEQ ID NO: 151 | SEQ ID NO: 152 |

### EXAMPLE 9 Production of the human antibody by genetically modification

(1) Construction of the recombinant human IgG-expressing plasmid
   With reference to the database information, a sense primer that contains ATG, the translation initiation codon of the human IgG heavy chain gene and an antisense primer that contains a translation stop codon are prepared, and using HumanSpleen 5'-Stretch cDNA Library (made by Clonetech Inc.) as a template, PCR is performed. The amplified DNA fragment of the human IgG gene is integrated into pT7Blue (Novagen) to confirm the base sequence. With an appropriate restriction enzyme that does not cleave an internal sequence of the human IgG gene, the human IgG gene is excised from the pTBlue. Then the gene is inserted into the cloning site of pCAGGS, the expression vector to construct a human IgG heavy chain-expressing plasmid. In addition, construction of a human IgG light chain-expressing plasmid is performed in a similar manner.
(2) Cloning of the antibody gene
   By culturing hybridoma #2-6, cells are prepared. After washing the obtained cells with D-PBS (Sigma), total RNA is isolated and purified using TRIzol Reagent (Invitrogen). Next, using Oligo-dT primer and SuperScriptII system (Invitrogen), single-stranded cDNA is synthesized. PCR primers to amplify heavy chain and light chain variable region are synthesized, and using the single-stranded cDNA derived from the hybridoma as a template, PCR is done. The amplified DNA fragment is integrated into pT7Blue (Novagen) to confirm the base sequence.
(3) Construction of the human-human chimeric antibody-expressing plasmid
   Heavy chain variable region is excised using the suitable restriction enzyme which can excise the region of the human IgG gene and is replaced with the heavy chain variable region derived from the hybridoma. At this time, the DNA fragment of the heavy chain variable region derived from the hybridoma is amplified by PCR using the primer which contains the same sequence as that of the restriction enzyme cleavage site to be inserted. Recombinant human IgG-expressing plasmid having a light chain variable region derived from the hybridoma is constructed in the similar way in the case of the heavy chain.
(4) Selection of the human-human chimeric antibody-producing clone
   Desired antibody, which is introduced into CHO (CCL-61) or SP2/0-ag14 (ATCC CRL1581) and expressed in culture supernatant, is selected by a binding activity to GPVI. Specifically, first, 4 µg each of the chimeric antibody heavy chain-expressing plasmid, the light chain-expressing plasmid and pSV2-neo (total 12 µg)is mixed with 60 µgL of FuGENE6 (Roche diagnostics), the transfection reagents, and stood for a while. Next, to a cell culture fluid that has been cultivated to semi-confluentit state in the 150 cm² culture flask, the mixture of the plasmids and FuGENE is added. After cultivation for two or three days, the cells are seeded on 96-well microtiter plate at the density of 0.7 cells/well, and in the medium containing 0.2 mg/ml G-418, further cultivation is performed for two or three weeks. From the well, in which a colony is formed, culture supernatant is collected to get the clone having the binding activity to GPVI by assaying the activity using EIA.
(5) Production of the human-human chimeric antibody
   The human- human chimeric antibody-producing clone is cultured with the culture medium supplemented with serum, and after replacing the medium with serum-free culture medium (Hybridoma-SFM, Invitrotec) at the step of confluence, a more two-day cultivation is performed. The obtained culture supernatant is purified with the Protein A column (Prosep-A, Milipore) to get a purified chimeric antibody.

### EXAMPLE 10 Preparation of IgG4 and Fab of hybridoma-derived anti-GPVI antibody

(1) Materials and equipments
Materials and equipments were shown below.
·Primers (synthesized by SIGMA Genosys Japan) HV3-11-a 5' GGAGTTTCCATTCGGTGATCAG 3' (SEQ ID NO: 153) IGLV2-14-a 5' GTGCTGGGGTCTCAGGAGGCAG 3' (SEQ ID NO: 154) IgL-a 5' CTATGAACATTCTGTAGGGGC 3' (SEQ ID NO: 155) IgL-b 5' TGCAGCTCTAGTCTCCCGTGG 3' (SEQ ID NO: 156) IgM-1 5' GGGAAGGAAGTCCTGTGCGA 3' (SEQ ID NO: 157) IGLV1-51-a 5' CAGCTGTGAGCGCAGAAGGCAG 3' (SEQ ID NO: 158) IGLV1-51-b 5' TCGGGACAATCTTCATCATG 3' (SEQ ID NO: 159) IgG4-a 5' CGGTCACATGGCACCACCTCT 3' (SEQ ID NO: 160) 
IgG4-c 5' TGGACAAGAGAGTTGAGTCCAAATATGGTC 3' (SEQ ID NO:
161)
 
IgG4-d 5' GACCATATTTGGACTCAACTCTCTTGTCCA 3' (SEQ ID NO:
162)
 
IgG4-f 5' GCCCATCATGCCCAGCACCTGAGTTCCTGG 3' (SEQ ID NO:
163)
 
IgG4-g 5' CCAGGAACTCAGGTGCTGGGCATGATGGGC 3' (SEQ ID NO:
164)
 
IgG4-h 5' TCTCCAAAGCCAAAGGGCAGCCCCGAGAGC 3' (SEQ ID NO:
165)
 
IgG4-I 5' GCTCTCGGGGCTGCCCTTTGGCTTTGGAGA 3' (SEQ ID NO:
166)
 IgG4-j 5' CCCTGGCACTCATTTACCCAG 3' (SEQ ID NO: 167) IgG4-k 5' GACGGGGTACGTGCCAAGCATCCT 3' (SEQ ID NO: 168) 
IgG4-m 5' AGCGCTAGCACCAAGGGCCCATCCGTCTTC 3' (SEQ ID NO:
169)
 
IgG4-n 5' AGATCTTCATGGGGGACCATATTTGGACTC 3' (SEQ ID NO:
170)
 
IgG4-t 5' TTAATGATGATGATGATGATGTGGGGGACC 3' (SEQ ID NO:
171)
 M4 5' GTTTTCCCAGTCACGACG 3' (SEQ ID NO: 172) 
HchainREV-ScaI 5' GGGCGGAGTACTGGAGACGGTGACC 3' (SEQ ID NO:
173)
 
HchainEco47NheI 5' CCCCCGCTAGCGCTGGAGACGGTGACC 3' (SEQ ID
NO: 174)
 
mIgG2c-a 5' GGATCCAAAACAACAGCCCCATCGGTCTAT 3' (SEQ ID NO:
175)
 
mIgG2c-b 5' TGGACAAGAAAATTGAGCCCAGAGTGCCCA 3' (SEQ ID NO:
176)
 
mIgG2c-c 5' TGGGCACTCTGGGCTCAATTTTCTTGTCCA 3' (SEQ ID NO:
177)
 
mIgG2c-d 5' GTCCCCCATGCGCAGCTCCAGACCTCTTGG 3' (SEQ ID NO:
178)
 
mIgG2c-e 5' CCAAGAGGTCTGGAGCTGCGCATGGGGGAC 3' (SEQ ID NO:
179)
 
mIgG2c-f 5' TCTCAAAACCCAGAGGGCCAGTAAGAGCTC 3' (SEQ ID NO:
180)
 
mIgG2c-g 5' GAGCTCTTACTGGCCCTCTGGGTTTTGAGA 3' (SEQ ID NO:
181)
 
mIgG2c-h 5' AGATCTTCATTTACCCAGAGACCGGAGAT 3' (SEQ ID NO:
182)
·Enzyme for PCR reaction
   Ex Taq (TAKARA BIO INC.)
   As the 10X reaction buffer and dNTP mixture, the ones attached to the enzyme were used.
·Genomic DNA
   HeLa genome lot. N34707-1 (BD Biosciences Clontech)
   Mouse genomic DNA (extracted from tail of B6 mouse)
·The PCR equipment
   DNA Engine (MJ RESEARCH, INC.)
·Agarose gel electophoresis
   SeaKem GTG Agarose (TAKARA BIO INC.)
   Submarine type electrophoresis apparatus (ADVANCE)
   50X TAE (2 mol/L Tris-acetate, 0.05 mol/L EDTA) (NIPPON GENE)
   Molecular weight marker (Sty I digested λ DNA fragments)
· DNA fragment extraction kit from the gel
   QIAEX II (QIAGEN K.K.)
· Expression vector for mammalian cells
   pEF2cew (The improved expression vector, wherein CMV enhancer is added upstream EF promoter of the pEF-BOS)
· Vector for the TA cloning and the reagents for ligation
   pT7BlueT (NOVAGEN)
   TaKaRa Ligation Kit ver.2 (TAKARA BIO INC.)
· E. coli Competent cell JM109 (TAKARA BIO INC.)
· Plasmid purification kit (QIAGEN K.K.)
· Kit for sequencing and the analyzer
   DYEnamic ET Terminator Cycle Sequencing Premix Kit lot.1767 (Amersham Biosciences)
   ABI3100 genetic analyzer (Applied Biosystems)
· Culture medium
   The Dulbecco MEM culture midium (Sigma)
·Reagents for transfection
   FuGENE6 (Roche Diagnostics)

(2) Experimental methods
Principal experimental methods used were shown below.
·PCR reaction
The reaction was performed according to the instructions attached to the enzyme. Example of the composition for the reaction mixture was shown below.

**Reaction mixture**

| | |
|---|---|
| TaKaRa Ex Taq (5 units/µL) | 0.25 µL |
| 10X Ex Taq Buffer | 5 µL |
| dNTP Mixture (2.5 mM each) | 4 µL |
| human kidney first strand cDNA | 1 µL |
| sense primer (10 pmol/µL) | 1 µL |
| anti-sense primer (10 pmol/µL) | 1 µL |
| H₂O | 37.75 µL |

(a) Agarose gel electophoresis
   Firstly, an agarose gel with the concentration of 0.8% was prepared. It was placed in the electrophoresis chamber that was filled with 1x TAE. After applying 5 µL of sample to well, the samples were electrophoresed at 135V for 15 minutes. After completion of the electrophoresis, the gel was stained with ethidium bromide. Further, a band was detected by irradiating an UV. In addition, a molecular weight marker was electrophoresed at the same time.
(b) Extraction of DNA fragment from the gel
   Desired band was excised using razor. DNA was extracted from the gel segment using QIAEX II Kit. The procedures were according to the instruction manual attached. The extracted DNA fragment was dissolved in 20 µL of the sterilized water.
(c) Ligation reaction
   The extracted DNA fragment, 1 µL and 1 µL of the cloning vector pT7BlueT, and 2 µL of solution I of the ligation kit ver.2 were mixed and stood at room temperature for 15 minutes.
(d) Transformation of Escherichia coli
   Competent Escherichia coli, 50 µL was thawed on ice. To the cells, 4 µL of the ligation product was adde. The mixture was directly stood on ice for 30 minutes. Heat shock at 42°C for 45 seconds was given to the cells. The cells were plated onto LB plate containing 50 µg/mL ampicillin at the final concentration and incubated at 37°C for overnight.
(e) Purification of the plasmid and sequencing reaction
   The respective procedures were according to the instruction manuals of the kits.
(f) Transfection
   Transfection was performed according to the instruction manual of FuGENE6. In case of the 6-well plate, in the preceding day of transfection at the density of 1.5x10⁵ cells/mL, 2 mL of the cells were inoculated in each well. The next day, after admixing 3 µL of FuGENE6 and 1 µg of the expression vector with 97 µL of the Dulbecco MEM culture medium and standing for 15 minutes or more, transfection was performed by dropping the admixture into 2 mL of serum-free Dulbecco MEM culture medium and replacing with culture fluid.

(3) Cloning of the gene encoding a heavy chain variable region and a light chain
1) From whole RNA extracted from each hybridoma, by the reverse transcription reaction using an oligo dT primer, a single-stranded cDNA.was synthesized. Next, by each reaction which is shown below, gene fragments encoding a heavy chain variable region and a light chain were amplified, respectively.
   a) By the PCR reaction using single-stranded cDNA of #2-4 as a template and primers (HV3-11-a and IgM-1), cDNA encoding a heavy chain variable region was specifically amplified. On the other hand, the first PCR reaction using primers (IGLV1-51-a and IgL-b) was performed. Then by performing the second PCR reaction (nested-PCR) using the first PCR product as a template and primers (IGLV1-51-b and IgL-a), cDNA encoding a light-chain was amplified.
   b) By the PCR reaction using single-stranded cDNA of #2-6 as a template and primers (HV3-11-a and IgM-1), cDNA encoding a heavy chain variable region was specifically amplified. In a similar way, the PCR reaction using primers (IGLV2-14-a and IgL-b) was performed to amplify cDNA encoding a light chain.
2) Each amplified fragment was cloned into pT7-BlueT vector, and the sequences were confirmed. The plasmid having a heavy chain variable region of #2-4, and the plasmid having a light chain of #2-4 were designated pT7-#2-4H and , a light-chain pT7-#2-4λ, respectively. In the case of #2-6, they were designated pT7-#2-6H and pT7-#2-6λ, respectively in a similar way.

(4) Cloning of the gene encoding a heavy chain constant region (Cγ4)
Using HeLa genomic DNA as a template and the following primer pair, PCR reaction was performed. As a result, gene regions encoding respective domains such as the CH1 domain in IgG4-a and IgG4-d, the hinge region in IgG4-c and IgG4-g, the CH2 domain in IgG4-f and IgG4-i, and the CH3 domain in IgG4-h and IgG4-k, were amplified. Next, the four amplified product was mixed and PCR using primers IgG4-m and IgG4-j was performed. Then the amplified product where each domain was connected was obtained. The amplified product was cloned into the pT7-BlueT vector, and the sequence encoding a heavy chain constant region (Cγ4) was confirmed. The clone was designated pTK-2232.
(5) Construction of a heavy chain-expressing plasmid for the antibody expression
The gene regions encoding a heavy chain variable region of the pT7-#2-4H and the pT7-#2-6H was amplified by the PCR reaction using primers (M4 and HchainREV-ScaI). Then the amplified products were cleaved with the restriction enzymes Eco RI and Sca I to prepare fragment A.
On the other hand, the pTK-2232 was cleaved with the restriction enzymes Eco 47III and Bam HI to prepare gene fragment B encoding a heavy chain constant region (Cγ4).
These fragments were ligated downstream EF promoter of the expression vector pEF2cew, which was prepared by cleaving with Eco RI and Bam HI, in the direction of fragment A + fragment B to construct heavy chain-expressing plasmids. After confirmation of the sequence, the plasmids were designated pTK-#2-4γ and pTK-#2-6γ.
(6) Construction of the heavy chain-expressing plasmid for the Fab expression
1) Construction of the plasmid which expresses only Fab heavy chain
   The gene regions encoding a heavy chain variable region of the pT7-#2-4H and the pT7-#2-6H was amplified by the PCR reaction using primers (M4 and HchainEco47NheI). Then the amplified products were cleaved with the restriction enzymes Eco RI and Nhe I to prepare fragment C.
   On the other hand, by perfoming the PCR reaction using pTK-2232 as a template and primers (IgG4-m and IgG4-n), the CH1 gene fragment having a stop codon immediately after the CH1 domain was amplified. The amplified fragment was cloned into the pT7-BlueT vector to construct pT7-IgG4mn. The pT7-IgG4mn was cleaved with the restriction enzymes Nhe I and Bgl II to prepare fragment D.
   These fragments were ligated downstream EF promoter of the expression vector pEF2cew, which was prepared by cleaving with Eco RI and Bam HI, in the direction of fragment C + fragment D to construct plasmids that express only Fab heavy chain. After confirmation of the sequence, the plasmids were designated pTK-#2-4Fab and pTK-#2-6Fab.
2) Construction of the plasmid which expresses the Fab heavy chain having histidine tag (His-tag) at the C-terminus (Fab-His)
   By perfoming the PCR reaction using pTK-2232 as a template and primers (IgG4-m and IgG4-t), the CH1 gene fragment having a His-tag immediately after the CH1 domain was amplified. The amplified fragment was cloned into the pT7-BlueT vector to construct pT7-IgG4mt. The pT7-IgG4mt was cleaved with the restriction enzymes Nhe I and Bam HI to prepare fragment E.
   These fragments were ligated downstream EF promoter of the expression vector pEF2cew, which was prepared by cleaving with Eco RI and Bam HI, in the direction of fragment C + fragment E to construct plasmids that express the Fab heavy chain having His-tag at the C-terminus. After confirmation of the sequence, the plasmids were designated pTK-#2-4Fab-His and pTK-#2-6Fab-His.

(7) Construction of the light chain-expressing plasmid
The plasmids having the light chain genes (pT7-#2-4λ and pT7-#2-6λ) were cleaved with suitable restriction enzymes such as Eco RI and Xba I, which cannot cleave the light chain region, to prepare fragment F.
The fragment F was ligated downstream EF promoter of the expression vector pEF2cew, which was prepared by cutting with the same restriction enzymes to construct the light chain-expressing plasmid. After confirmation of the sequence, the plasmids were designated pTK-#2-4λ and pTK-#2-6λ.
(8) Expression of the recombinant antibody and Fab
1) The COS-1 cells were cultured by passage through with a Dulbecco MEM culture medium containing 10% fetal bovine serum, and in the preceding day of transfection at the density of 1.5x10⁵ cells/mL, the cells were inoculated in a culture vessel. The next day, after admixing the heavy chain (or Fab)-expressing plasmid and the light chain-expressing plasmid with the transfection reagents (FuGENE6, Roche Diagnostics) in an adequate amount, cotransfection was performed by dropping the admixture into serum-free Dulbecco MEM culture medium and replacing with culture fluid.
   As a result, when the pTK-#2-4γ and the pTK-#2-4λ were cotransfected, it was possible to make the R#2-4 antibody, which is a recombinant IgG4 antibody, secrete into the culture fluid. In a similar way, when the pTK-#2-6γ and the pTK-#2-6λ were used, it was possible to make the R#2-6 antibody secrete into the culture fluid. When the pTK-#2-4Fab and the pTK-#2-4λ, or the pTK-#2-6Fab and the pTK-#2-6λ were used, it was possible to make the recombinant Fabs (R#2-4Fab and R#2-6Fab) secrete into the culture fluid. When the pTK-#2-4Fab-His and the pTK-#2-4λ, or the pTK-#2-6Fab-His and pTK-#2-6λ were used, it was possible to make the recombination Fabs having His-tag (R#2-4Fab-His and R#2-6Fab-His) secrete into the culture fluid.
2) In the presence of 5% CO₂, the cells were cultured at 37°C for two or three days and the culture fluid was collected.

(9) Purification of the recombinant antibody
[Purification of the recombinant IgG antibody]
Purification of the recombinant IgG4 antibodies (R#2-4 and R#2-6 antibodies) from the culture fluid was performed using Prosep-A column (MILLIPORE), and after dialysis against PBS (pH 7.4), the concentration was calculated from the absorbance at 280 nm.
[Purification of the recombinant Fab]
1) Purification of the recombinant Fab molecules (R#2-4 and R#2-6 Fabs) from the culture fluid was performed using anti-human lambda light chain antibody column (homemade).
2) Purification of the recombinant Fab molecules having His-tag (R#2-4Fab-His and R#2-6Fab-His) from the culture fluid was achieved by the first purification using the Hi-Trap Chelating HP (Amersham) followed by the second purification using the anti-human lambda light chain antibody column.
3) After purification, all Fabs were dialyzed against PBS (pH 7.4), and the concentration was calculated from the absorbance at 280 nm. In addition, Fab for an inhibition experiment of platelet aggregation was dialyzed against the physiological saline.

### EXAMPLE 11 Preparation of a human GPVI-mouse Fc fusion protein

(1) Construction of the GPVI-mFc fusion protein-expressing plasmid
   1) Using mouse genomic DNA as a template and the following primer pair, PCR reaction was performed. As a result, gene regions encoding respective domains of mouse immunoglobulin (mIgG2c) heavy chain constant region such as the CH1 domain in mIgG2c and mIgG2c-c, the hinge region in mIgG2c-b and mIgG2c-e, the CH2 domain in mIgG2c-d and mIgG2c-g, and the CH3 domain in mIgG2c-f and mIgG2c-h, were amplified. Next, the four amplified product was mixed and PCR using primers mIgG2c-a and mIgG2c-h was performed. Then the amplified product where each domain was connected was obtained. The amplified product was cloned into the pT7-BlueT vector, and the sequence encoding heavy chain constant region (Cγ2c) was confirmed. The clone was designated pT7-mIgG2c.
   2) From the pT7-mIgG2c, a gene fragment encoding mouse Fc region was excised using restriction enzymes Bam HI and Kpn I to prepare fragment H. On the other hand, from pCAGGS-GPVI-Fc plasmid, a gene fragment encoding extracellular domain of the human GPVI was excised using restriction enzymes Xba I and Bgl II to prepare fragment I. These fragments were ligated downstream EF promoter of the expression vector pEF2cew, which was prepared by cleaving with Xba I and Kpn I, in the direction of fragment H + fragment I to construct a plasmid that expresses human GPVI and mouse Fc fusion protein (GPVI-mFc). After confirmation of the sequence, the plasmid was designated pTK-2249.
(2) Expression and purification of the GPVI-mFc fusion protein
   1) The COS-1 cells were cultured by passage through with a Dulbecco MEM culture medium containing 10% fetal bovine serum, and in the preceding day of transfection at the density of 1.5x10⁵ cells/mL, the cells were inoculated in a culture vessel. The next day, after admixing the pTK-2249 with the transfection reagents (FuGENE6, Roche Diagnostics) in an adequate amount, transfection was performed by dropping the admixture into serum-free Dulbecco MEM culture medium and replacing with culture fluid.
   2) In the presence of 5% CO₂, the cells were cultured at 37°C for two or three days and the culture fluid was collected.
   3) Purification of the GPVI-mFc fusion protein from the culture fluid was performed using Prosep-A column (MILLIPORE), and after dialysis against PBS (pH 7.4), the concentration was calculated from the absorbance at 280 nm.

### EXAMPLE 12 Assay for the binding activity to GPVI measurement of the various anti-GPVI antibodies (Cell cytometry)

From a blood collected from normal subject, a platelet fraction was prepared by centrifugation. The platelet, 5x10⁶, was suspended in 50 µL of PBS- containing 5% human plasma and 0.5% inactivated fetal bovine serum (FBS). After adding 2 µg of the recombinant human anti-GPVI antibody (IgG4) with all kinds that were prepared in EXAMPLE 10, incubation at room temperature for one hour was performed. After washing twice with 1 mL of 0.5% inactivated FBS containing PBS-, the platelet was resuspended in 50 µL of 0.5% inactivated FBS containing PBS-. To the suspension, 1 µg of FITC-labeled anti-human IgG antibody was added, and incubation at room temperature for one hour was done. After washing twice with 1 mL of 0.5% inactivated FBS containing PBS-, an FITC positive platelet-count was measured in Cytometric FC500 (Beckman Coulter).

As a result, equal to or more than 90% of the platelet, which reacted with R#2-6 antibody, and 68% of the platelet, which reacted with R#2-4 antibody, were FITC positive cells.

In addition, Fig. 4 shows that the recombinant human anti-GPVI antibodies (R#2-4 and R#2-6) bind to the platelet which was prepared from the human peripheral blood. In Fig. 4, the white-colored histogram shows the fluorescence intensity distribution when the human whole IgG as a negative control was reacted. The gray-colored histogram shows the fluorescence intensity distribution when R#2-4 or R#2-6 antibody was reacted.

### EXAMPLE 13 Assay for the binding activity to GPVI measurement of the various anti-GPVI antibodies (ELISA method)

GPVI-hFc chimeric protein or GPVI-mFc chimeric protein was solidified on 96-well plate for ELISA by preparing each protein for 3 µg/mL with PBS-, adding 50 µL/well to the plate and incubating at 37°C for two hours. After five times washing with 400 µL/well of PBS-, the well was blocked at room temperature for one hour with 2% StabiliGuard/PBS-. The recombinant human anti-GPVI antibodies (IgG4) with all kinds which was prepared in EXAMPLE 10 were added to the GPVI-Fc solidified plate at the concentration of 10 µg/mL and was reacted at room temperature for two hours. After washing three times with 0.05% Tween20 containing PBS-, and twice with PBS-, a second antibody (HRP-labeled anti-human κ-light chain antibody, or HRP-labeled anti-human λ-light chain antibody), which were diluted respectively by 1000 times or 2000 times with PBS-, was added to each well and incubated at room temperature for two hours. After washing three times with 0.05% Tween20 containing PBS-, and twice with PBS-, TMB solution was added. Color was developed at room temperature for 20 minutes. Development was terminated by adding 1 M sulfuric acid, and an absorbance at 450 nm was measured.

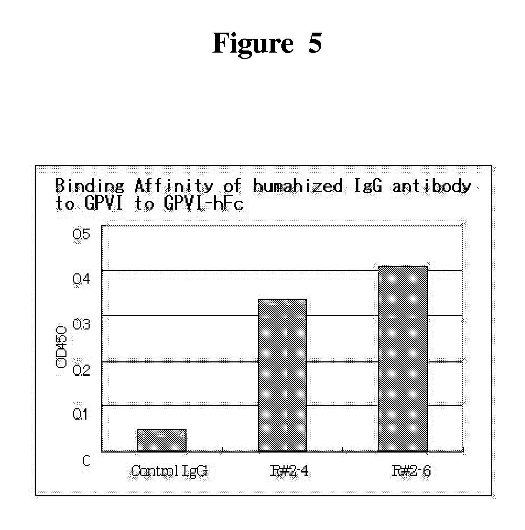

As a result, while the human whole IgG as a negative control could hardly bind to GPVI-hFc at all, R#2-4 and R#2-6 antibodies exhibited a remarkable binding activity to GPVI-hFc at the concentration of 10 µg/mL (Figure 5). In addition, R#2-6 and R#2-4 antibodies showed an affinity to GPVI-mFc.

### EXAMPLE 14 The independent influence of the anti-GPVI antibody on the whole blood and the platelet

(1) The influence of the anti-GPVI antibody in blood clot formation using the whole blood
   To 2 ml of the blood collected from normal subject by using anti-thrombin agent, a various GPVI antibody was added to make a final concentration 30 µg/mL. Subsequently, incubation at 37°C for 3 hours was performed. Then, blood clot formation was confirmed by visual check. Since the known anti-GPVI antibody such as an IgG derived from a patient with autoimmune thrombocytopenia can solely induce a platelet aggregation (non-patent document 2), it is thought that in this system, coagulation system is excessively activated and blood clot formation is induced. However, like the case of the control IgG addition, in neither of R#2-4 and R#2-6 antibody addition, blood clot formation was detected at all.
(2) The influence of the anti-GPVI antibody on the platelet
   According to EXAMPLE 6, using PRP, a platelet aggregation induction with the sole antibody was measured. As a result, in the concentration of 100 µg/mL, both R#2-4 and R#2-6 antibodies cannot induce a human platelet aggregation solely.

### EXAMPLE 15 The effect of the humanized anti-GPVI antibody on the platelet response toward collagen

To 2 ml of the blood collected from normal subject by using anti-thrombin agent, a various GPVI antibody was added. Subsequently, incubation at 37°C for 3 hours was performed. After completion of the incubation, the platelet was isolated to prepare 3x10⁸ platelets/ml. To this, CaCl₂ solution was added to become 1 mM of the final concentration. Then incubation at 37°C for 3 minutes was done with stirring. To the solution, collagen solution was added to become 1-2 µg/mL of the final concentration. Turbidity was measured with the platelet aggregation analyzer (AC Medical Inc., MCM hematoracer 801).

As a result, with respect to the platelet that was treated with 30 µg/mL of R#2-6 or R#2-4 antibody, the remarkable decline of the platelet agglutinability toward the collagen was detected.

**Table 8**

| | The antibody concentration | The maximum-agglutination-rate |
|---|---|---|
| Control | - | 70% |
| R#2-4 | 30 µg/mL | 10% |
| R#2-6 | 30 µg/mL | 2% |

### INDUSTRIAL APPLICABILITY

Since the antibody of the present invention decreases a platelet-agglutinability by specifically binding to GPVI on the human platelet, it can be used as a medicament such as an anti-platelet agent. In addition, the human antibody of the present invention is useful regarding no immunogenicity, which the heteroantibody, the chimeric antibody, and the humanized antibody have, even if it is administered to the human as a medicament. The antibody of the present invention, which does not induce a human platelet agglutination independently, can directly be administered as a medicament without a treatment, for example, a processing to prepare Fab. The antibody of the present invention can suppress a platelet aggregation mediated by the collagen at less dosage than the existing antibody and has efficacy as a medicament at less amount.

SEQUENCE LISTING

<110> Mochida Pharmaceutical, Co., Ltd.

<120> Human antiplatelet membrane glycoprotein monoclonal antibody

<130> C05-0095

<150> PCT/JP04/010596
<151> 2004-07-20

<150> JP 2003-199192
<151> 2003-07-18

<160> 182

<170> PatentIn version 3.1

<210> 1
<211> 5
<212> PRT
<213> Homo sapiens

<400> 1

Asp Tyr Tyr Met Ser
1               5

<210> 2
<211> 17

<212> PRT
<213> Homo sapiens

<400> 2

Tyr Ile Thr Ser Ser Ser Ser Tyr Thr Asn Tyr Ala Asp Ser Val Lys
1               5                   10                  15
Gly

<210> 3
<211> 15
<212> PRT
<213> Homo sapiens

<400> 3

Asp Arg Ala Val Arg Gly Val Ile Ile Ile Arg Pro Pro Asp Tyr
1               5                   10                  15

<210> 4
<211> 14
<212> PRT
<213> Homo sapiens

<400> 4

Thr Gly Thr Ser Ser Asp Val Gly Gly Tyr Asn Tyr Val Ser
1               5                   10

<210> 5
<211> 7
<212> PRT
<213> Homo sapiens

<400> 5

Asp Val Ser Asn Arg Pro Ser
1               5

<210> 6
<211> 10
<212> PRT
<213> Homo sapiens

<400> 6

Ser Ser Tyr Thr Ser Ser Ser Thr Leu Val
1               5                   10

<210> 7
<211> 5
<212> PRT
<213> Homo sapiens

<400> 7

Ser Tyr Ala Met Ser
1               5

<210> 8
<211> 17
<212> PRT
<213> Homo sapiens

<400> 8

Ala Ile Ser Gly Ser Gly Gly Ser Thr Tyr Tyr Ala Asp Ser Val Lys
1               5                   10                  15
Gly

<210> 9
<211> 9
<212> PRT
<213> Homo sapiens

<400> 9

His Phe Ile Leu Thr Gly Tyr His Tyr
1               5

<210> 10
<211> 13
<212> PRT
<213> Homo sapiens

<400> 10

Scr Gl y Scr Scr Scr Asn Ilc Gl y Asn Asn Tyr Val Scr
1               5                   10

<210> 11
<211> 7
<212> PRT
<213> Homo sapi ens

<400> 11
   Asp Asn Asn Lys Arg Pro Ser
   1               5

<210> 12
<211> 11
<212> PRT
<213> Homo sapi ens

<400> 12
   Gly Thr Trp Asp Ser Ser Leu Ser Ala Gly Val
   1               5                   10

<210> 13
<211> 124
<212> PRT
<213> Homo sapi ens

<400> 13

Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp Tyr
            20                  25                  30
Tyr Met Ser Trp Ile Arg Gln Ala Pro Gly Lys Gly Leu Glu Trp Val
        35                  40                  45
Ser Tyr Ile Thr Ser Ser Ser Ser Tyr Thr Asn Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Arg Ala Val Arg Gly Val Ile Ile Ile Arg Pro Pro Asp
            100                 105                 110
Tyr Trp Gly Gln Gly Thr Leu Val Thr Val Ser Ser
        115                 120

<210>  14
<211>  126
<212>  PRT
<213>  Homo sapiens

<400>  14

Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45
Met Ile Tyr Asp Val Ser Asn Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Thr Ser Ser
                85                  90                  95
Ser Thr Leu Val Phe Gly Gly Gly Thr Lys Leu Thr Val Leu Gly Gln
            100                 105                 110
Pro Lys Ala Ala Pro Ser Val Thr Leu Phe Pro Pro Ser Ser
        115                 120                 125

<210> 15
<211> 137
<212> PRT
<213> Homo sapiens

<400> 15

Met Glu Phe Gly Leu Ser Trp Leu Phe Leu Val Ala Ile Leu Lys Gly
1               5                   10                  15
Val Gln Cys Glu Val Gln Leu Leu Glu Ser Gly Gly Gly Leu Val Gln
            20                  25                  30
Pro Gly Gly Ser Leu Arg Leu Ser Cys Ala Ala Ser Gly Phe Thr Phe
        35                  40                  45
Ser Ser Tyr Ala Met Ser Trp Val Arg Gln Ala Pro Gly Lys Gly Leu
    50                  55                  60
Glu Trp Val Ser Ala Ile Ser Gly Ser Gly Gly Ser Thr Tyr Tyr Ala

65                  70                  75                  80
Asp Ser Val Lys Gly Arg Phe Thr Ile Ser Arg Asp Asn Ser Lys Asn
                85                  90                  95
Thr Leu Tyr Leu Gln Met Asn Ser Leu Arg Ala Glu Asp Thr Ala Val
            100                 105                 110
Tyr Tyr Cys Ala Asn His Phe Ile Leu Thr Gly Tyr His Tyr Trp Gly
        115                 120                 125
Gln Gly Thr Leu Val Thr Val Ser Ser
    130                 135

<210> 16
<211> 129
<212> PRT
<213> Homo sapiens

<400> 16

Met Thr Cys Ser Pro Leu Leu Leu Thr Leu Leu Ile His Cys Thr Gly
1               5                   10                  15
Ser Trp Ala Gln Ser Val Leu Thr Gln Pro Pro Ser Val Ser Ala Ala
            20                  25                  30
Pro Gly Gln Lys Val Thr Ile Ser Cys Ser Gly Ser Ser Ser Asn Ile
        35                  40                  45
Gly Asn Asn Tyr Val Ser Trp Tyr Gln Gln Leu Pro Gly Thr Ala Pro
    50                  55                  60
Lys Leu Leu Ile Tyr Asp Asn Asn Lys Arg Pro Ser Gly Ile Pro Asp
65                  70                  75                  80
Arg Phe Ser Gly Ser Lys Ser Gly Thr Ser Ala Thr Leu Gly Ile Thr
                85                  90                  95

Gly Leu Gln Thr Gly Asp Glu Ala Asp Tyr Tyr Cys Gly Thr Trp Asp
            100                 105                 110
Ser Ser Leu Ser Ala Gly Val Phe Gly Gly Gly Thr Lys Leu Thr Val
        115                 120                 125
Leu

<210> 17
<211> 15
<212> DNA
<213> Homo sapiens

<400> 17
gactactaca tgagc 15

<210> 18
<211> 51
<212> DNA
<213> Homo sapiens

<400> 18
tacattacta gtagtagtag ttacacaaac tacgcagact ctgtgaaggg c 51

<210> 19
<211> 45
<212> DNA
<213> Homo sapiens

<400> 19
gatcgagcgg ttcggggagt tattataatc cgcccgccag actac 45

<210> 20
<211> 42
<212> DNA
<213> Homo sapiens

<400> 20
actggaacca gcagtgacgt tggtggttat aactatgtct cc 42

<210> 21
<211> 21
<212> DNA
<213> Homo sapiens

<400> 21
gatgtcagta atcggccctc a 21

<210> 22
<211> 30
<212> DNA
<213> Homo sapiens

<400> 22
agctcatata caagcagcag cactctggta 30

<210> 23
<211> 15
<212> DNA

<213>  Homo sapiens

<400>  23
agctatgcca tgagc                                                        15

<210>  24
<211>  51
<212>  DNA
<213>  Homo sapiens

<400>  24
gctattagtg gtagtggtgg tagcacatac tacgcagact ccgtgaaggg c                51

<210>  25
<211>  27
<212>  DNA
<213>  Homo sapiens

<400>  25
cactttattt tgactggtta tcactac                                           27

<210>  26
<211>  39
<212>  DNA
<213>  Homo sapiens

<400>  26
tctggaagca gctccaacat tgggaataat tatgtatcc                              39

<210> 27
<211> 21
<212> DNA
<213> Homo sapiens

<400> 27
gacaataata agcgaccctc a 21

<210> 28
<211> 33
<212> DNA
<213> Homo sapiens

<400> 28
ggaacatggg atagcagcct gagtgctggg gtg 33

<210> 29
<211> 372
<212> DNA
<213> Homo sapiens

<400> 29
gaggtgcagc tggtggagtc tgggggaggc ttggtcaagc ctggagggtc cctgagactc 60
tcctgtgcag cctctggatt caccttcagt gactactaca tgagctggat ccgccaggct 120
ccagggaagg ggctggagtg ggtttcatac attactagta gtagtagtta cacaaactac 180
gcagactctg tgaagggccg attcaccatc tccagagaca acgccaagaa ctcactgtat 240
ctgcaaatga acagcctgag agccgaggac acggccgtgt attactgtgc gagagatcga 300
gcggttcggg gagttattat aatccgcccg ccagactact ggggccaggg aaccctggtc 360
accgtctcct ca 372

<210> 30

<211> 378

<212> DNA

<213> Homo sapiens

<400> 30

cagtctgccc tgactcagcc ggcctccgtg tctgggtctc ctggacagtc gatcaccatc 60

tcctgcactg gaaccagcag tgacgttggt ggttataact atgtctcctg gtaccaacaa 120

cacccaggca aagcccccaa actcatgatt tatgatgtca gtaatcggcc ctcaggggtt 180

tctaatcgct tctctggctc caagtctggc aacacggcct ccctgaccat ctctgggctc 240

caggctgagg acgaggctga ttattactgc agctcatata caagcagcag cactctggta 300

ttcggcggag ggaccaagct gaccgtccta ggtcagccca aggctgcccc ctcggtcact 360

ctgttcccac cctcctct 378

<210> 31

<211> 412

<212> DNA

<213> Homo sapiens

<400> 31

atggagtttg ggctgagctg gctttttctt gtggctattt taaaaggtgt ccagtgtgag 60

gtgcagctgt tggagtctgg gggaggcttg gtacagcctg gggggtccct gagactctcc 120

tgtgcagcct ctggattcac ctttagcagc tatgccatga gctgggtccg ccaggctcca 180

gggaaggggc tggagtgggt ctcagctatt agtggtagtg gtggtagcac atactacgca 240

gactccgtga agggccggtt caccatctcc agagacaatt ccaagaacac gctgtatctg 300

caaatgaaca gcctgagagc cgaggacacg gccgtatatt actgtgcgaa tcactttatt 360

ttgactggtt atcactactg gggccaggga accctggtca ccgtctcctc ag 412

<210> 32
<211> 387
<212> DNA
<213> Homo sapiens

<400> 32
atgacctgct cccctctcct cctcaccctt ctcattcact gcacagggtc ctgggcccag 60
tctgtgttga cgcagccgcc ctcagtgtct gcggccccag gacagaaggt caccatctcc 120
tgctctggaa gcagctccaa cattgggaat aattatgtat cctggtacca gcagctccca 180
ggaacagccc ccaaactcct catttatgac aataataagc gaccctcagg gattcctgac 240
cgattctctg gctccaagtc tggcacgtca gccaccctgg gcatcaccgg actccagact 300
ggggacgagg ccgattatta ctgcggaaca tgggatagca gcctgagtgc tggggtgttc 360
ggcggaggga ccaagctgac cgtccta 387

<210> 33
<211> 31
<212> DNA
<213> Artificial

<220>
<223> Primer

<400> 33
gctctagagc atgtctccat ccccgaccgc c 31

<210> 34
<211> 29
<212> DNA
<213> Artificial

<220>
<223> Primer

<400> 34
cgggatccgt tgcccttggt gtagtactg 29

<210> 35
<211> 32
<212> DNA
<213> Artificial

<220>
<223> Primer

<400> 35
aaaggatcca gatctaacga gcccaaatct tg 32

<210> 36
<211> 34
<212> DNA
<213> Artificial

<220>
<223> Primer

<400> 36
aaaggtaccc tatcatttac ccggagacag ggag 34

<210> 37
<211> 23
<212> DNA
<213> Artificial

<220>
<223> Primer

<400> 37
gaggtgcagc tggtggagtc tgg 23

<210> 38
<211> 24
<212> DNA
<213> Artificial

<220>
<223> Primer

<400> 38
tgaggagacg gtgaccaggg ttcc 24

<210> 39
<211> 23
<212> DNA
<213> Artificial

<220>
<223> Primer

<400> 39
gaggtgcagc tggtggagtc tgg 23

<210> 40
<211> 24
<212> DNA
<213> Artificial

<220>
<223> Primer

<400> 40
tgaggagacg gtgaccaggg ttcc 24

<210> 41
<211> 23
<212> DNA
<213> Artificial

<220>
<223> Primer

<400> 41
cagtctgccc tgactcagcc ggc 23

<210> 42
<211> 24
<212> DNA

<213>  Artificial

<220>
<223>  Primer

<400>  42
agaggagggt gggaacagag tgac                                              24

<210>  43
<211>  23
<212>  DNA
<213>  Artificial

<220>
<223>  Primer

<400>  43
cagtctgtct tgacgcagcc ggc                                               23

<210>  44
<211>  24
<212>  DNA
<213>  Artificial

<220>
<223>  Primer

<400>  44
agaggagggt gggaacagag tgac                                              24

<210> 45
<211> 19
<212> DNA
<213> Artificial

<220>
<223> Primer

<400> 45
gttttcccag tcacgacgt 19

<210> 46
<211> 20
<212> DNA
<213> Artificial

<220>
<223> Primer

<400> 46
ctaatacgac tcactatagg 20

<210> 47
<211> 5
<212> PRT
<213> Homo sapiens

<400> 47

Asn Tyr Ala Met Ala
1               5

<210> 48
<211> 17
<212> PRT
<213> Homo sapiens

<400> 48

Ala Ile Ser Val Ser Gly Thr Ser Thr Ala Tyr Ala Asp Ser Val Lys
1               5                   10                  15
Gly

<210> 49
<211> 12
<212> PRT
<213> Homo sapiens

<400> 49

Arg Gly Leu Pro His Pro Lys Tyr Phe Cys Asp Ser
1               5                   10

<210> 50
<211> 5
<212> PRT
<213> Homo sapiens

<400> 50

Ser Asn Tyr Met Ser
1               5

<210> 51
<211> 16
<212> PRT
<213> Homo sapiens

<400> 51

Val Ile Tyr Ser Gly Gly Ser Thr Tyr Tyr Ala Asp Ser Val Lys Gly
1               5                   10                  15

<210> 52
<211> 15
<212> PRT
<213> Homo sapiens

<400> 52

Leu Lys Ala Asp His Tyr Asp Ser Leu Ala Pro Asp Phe Asp Tyr
1               5                   10                  15

<210> 53
<211> 5
<212> PRT

<213> Homo sapiens

<400> 53

Ser Tyr Asp Met His
1               5

<210> 54
<211> 16
<212> PRT
<213> Homo sapiens

<400> 54

Ala Ile Gly Thr Ala Gly Asp Thr Tyr Tyr Pro Gly Ser Val Lys Gly
1               5                   10                  15

<210> 55
<211> 12
<212> PRT
<213> Homo sapiens

<400> 55

Ala Gly Lys Met Trp Trp Arg Gly Ala Phe Asp Ile
1               5                   10

<210> 56
<211> 5

<212> PRT
<213> Homo sapiens

<400> 56

Ser Tyr Ala Met Ser
1               5

<210> 57
<211> 17
<212> PRT
<213> Homo sapiens

<400> 57

Ala Ile Thr Gly Ser Gly Gly Thr Thr Tyr Tyr Ala Asp Ser Val Lys
1               5                   10                  15
Gly

<210> 58
<211> 12
<212> PRT
<213> Homo sapiens

<400> 58

Gly Gly Tyr Thr Ser Gly Asn Ser Tyr Phe Asp Tyr
1               5                   10

<210> 59
<211> 5
<212> PRT
<213> Homo sapiens

<400> 59

Thr Phe Tyr Ile His
1               5

<210> 60
<211> 17
<212> PRT
<213> Homo sapiens

<400> 60

Phe Ile Asn Pro Ser Gly Val Asn Thr Asn Tyr Ala Gln Lys Phe Gln
1               5                   10                  15
Asp

<210> 61
<211> 12
<212> PRT
<213> Homo sapiens

<400> 61

Asp Thr Arg Gly Trp Ser Leu Asn Gly Leu Asp Val
1               5                   10

<210> 62
<211> 5
<212> PRT
<213> Homo sapiens

<400> 62

Asp Tyr Ala Met His
1               5

<210> 63
<211> 17
<212> PRT
<213> Homo sapiens

<400> 63

Leu Ile Asn Gly Asp Gly Gly Gln Thr His Tyr Ala Asp Ser Val Lys
1               5                   10                  15
Gly

<210> 64
<211> 13
<212> PRT
<213> Homo sapiens

<400> 64

Gly Lys Arg Ser Gly Thr Tyr Tyr Asn Gly Leu Glu Tyr
1               5                   10

<210> 65
<211> 5
<212> PRT
<213> Homo sapiens

<400> 65

Asp Tyr Tyr Met Ser
1               5

<210> 66
<211> 17
<212> PRT
<213> Homo sapiens

<400> 66

Phe Ile Ser Ser Ser Ser Gly Tyr Thr Asp Tyr Ala Asp Ser Val Lys
1               5                   10                  15
Gly

<210> 67

<211> 9
<212> PRT
<213> Homo sapiens

<400> 67

Arg Ser Ser Gly Phe Pro Phe Asp Leu
1               5

<210> 68
<211> 5
<212> PRT
<213> Homo sapiens

<400> 68

Ser Asn Tyr Met Ser
1               5

<210> 69
<211> 16
<212> PRT
<213> Homo sapiens

<400> 69

Val Ile Tyr Ser Gly Gly Ser Thr Tyr Tyr Ala Asp Ser Val Lys Gly
1               5                   10                  15

<210> 70

<211> 7

<212> PRT

<213> Homo sapiens

<400> 70

Gly Arg Trp Ser Tyr Asp Tyr
1               5

<210> 71

<211> 5

<212> PRT

<213> Homo sapiens

<400> 71

Asp Tyr Tyr Met Ser
1               5

<210> 72

<211> 17

<212> PRT

<213> Homo sapiens

<400> 72

Tyr Ile Ser Ser Ser Ser Ser Tyr Thr Asn Tyr Ala Asp Ser Val Lys
1               5                   10                  15

Gly

<210> 73
<211> 13
<212> PRT
<213> Homo sapiens

<400> 73

Thr Leu Tyr Gly Ser Gly Ser Gly Asp Ala Phe Asp Ile
1               5                   10

<210> 74
<211> 5
<212> PRT
<213> Homo sapiens

<400> 74

Asp Tyr Gly Met Ser
1               5

<210> 75
<211> 17
<212> PRT
<213> Homo sapiens

<400> 75

Gly Ile Asn Trp Asn Gly Gly Ser Thr Gly Tyr Ala Asp Ser Val Lys
1               5                   10                  15
Gly

<210> 76
<211> 9
<212> PRT
<213> Homo sapiens

<400> 76

Ala Val Ala Thr Asp Ala Phe Asp Ile
1               5

<210> 77
<211> 5
<212> PRT
<213> Homo sapiens

<400> 77

Ser Tyr Trp Met His
1               5

<210> 78
<211> 17
<212> PRT

<213> Homo sapiens

<400> 78

Arg Ile Asn Ser Asp Gly Ser Ser Thr Ser Tyr Ala Asp Ser Val Lys
1               5                   10                  15
Gly

<210> 79
<211> 12
<212> PRT
<213> Homo sapiens

<400> 79

Asp Leu Ser Pro Gly Ser Gly Ser Pro Phe Asp Tyr
1               5                   10

<210> 80
<211> 7
<212> PRT
<213> Homo sapiens

<400> 80

Thr Ser Gly Val Gly Val Gly
1               5

<210> 81
<211> 16
<212> PRT
<213> Homo sapiens

<400> 81

Phe Ile Tyr Trp Asn Asp Asp Lys Arg Tyr Ser Pro Ser Leu Lys Ser
1               5                   10                  15

<210> 82
<211> 13
<212> PRT
<213> Homo sapiens

<400> 82

Arg Glu Ile Ala Ala Ala Gly Leu Tyr Ala Phe Asp Ile
1               5                   10

<210> 83
<211> 5
<212> PRT
<213> Homo sapiens

<400> 83

Asp Tyr Ala Met His
1               5

<210> 84
<211> 17
<212> PRT
<213> Homo sapiens

<400> 84

Leu Ile Ser Gly Asp Gly Gly Ser Thr Tyr Tyr Ala Asp Ser Val Lys
1               5                   10                  15
Gly

<210> 85
<211> 15
<212> PRT
<213> Homo sapiens

<400> 85

Gly Ser Tyr Asp Ser Ser Gly Tyr Tyr Pro Gly Ala Phe Asp Ile
1               5                   10                  15

<210> 86
<211> 5
<212> PRT
<213> Homo sapiens

<400> 86

Asp Tyr Gly Met Ser
1               5

<210> 87
<211> 17
<212> PRT
<213> Homo sapiens

<400> 87

Gly Ile Asn Trp Asn Gly Gly Ser Thr Gly Tyr Ala Asp Ser Val Lys
1               5                   10                  15
Gly

<210> 88
<211> 13
<212> PRT
<213> Homo sapiens

<400> 88

Gly Pro Thr Ile Ala Gly Tyr Tyr Tyr Gly Met Asp Val
1               5                   10

<210> 89
<211> 5
<212> PRT

<213>  Homo sapiens

<400>  89

Asn Tyr Ala Met His
1               5

<210>  90
<211>  17
<212>  PRT
<213>  Homo sapiens

<400>  90

Val Ile Ser Phe Asp Gly Arg Ser Lys Tyr Tyr Ala Asp Ser Val Arg
1               5                   10                  15
Gly

<210>  91
<211>  15
<212>  PRT
<213>  Homo sapiens

<400>  91

Glu Ile Gly Ala Ser Tyr Tyr Gly Ser Gly Gly Thr Pro Gly Tyr
1               5                   10                  15

<210>  92

<211> 5

<212> PRT

<213> Homo sapiens

<400> 92

Ser Tyr Tyr Trp Ser
1               5

<210> 93

<211> 16

<212> PRT

<213> Homo sapiens

<400> 93

Arg Ile Tyr Thr Ser Gly Ser Thr Asn Tyr Asn Pro Ser Leu Lys Ser
1               5                   10                  15

<210> 94

<211> 11

<212> PRT

<213> Homo sapiens

<400> 94

Asp Leu Ala Ala Arg Pro Asn Trp Phe Asp Pro
1               5                   10

<210> 95
<211> 5
<212> PRT
<213> Homo sapiens

<400> 95

Ser Tyr Ala Met Ser
1               5

<210> 96
<211> 17
<212> PRT
<213> Homo sapiens

<400> 96

Ala Ile Ser Gly Ser Gly Gly Ser Thr Tyr Tyr Ala Asp Ser Val Lys
1               5                   10                  15
Gly

<210> 97
<211> 24
<212> PRT
<213> Homo sapiens

<400> 97

Asn Leu Pro Ala Pro Gly Tyr Cys Ser Ser Thr Ser Cys Tyr Ala Leu

1               5                   10                  15
Tyr Tyr Tyr Tyr Gly Met Asp Val
            20

<210> 98
<211> 14
<212> PRT
<213> Homo sapiens

<400> 98

Thr Gly Thr Ser Ser Asp Ile Gly Ala Tyr Asp Phe Val Ser
1               5                   10

<210> 99
<211> 7
<212> PRT
<213> Homo sapiens

<400> 99

Asp Val Arg Asn Arg Pro Ser
1               5

<210> 100
<211> 10
<212> PRT
<213> Homo sapiens

<400> 100

Ser Ser Phe Thr Thr Ser Ser Val Trp Val
1               5                   10

<210> 101
<211> 14
<212> PRT
<213> Homo sapiens

<400> 101

Thr Gly Thr Ser Ser Asp Val Gly Gly Tyr Asn Tyr Val Ser
1               5                   10

<210> 102
<211> 7
<212> PRT
<213> Homo sapiens

<400> 102

Glu Val Ser Lys Arg Pro Ser
1               5

<210> 103
<211> 10
<212> PRT
<213> Homo sapiens

<400> 103

Ser Ser Tyr Ala Gly Ser Asn Met Gly Val
1               5                   10

<210> 104
<211> 7
<212> PRT
<213> Homo sapiens

<400> 104

Trp Ala Ser Thr Arg Glu Ser
1               5

<210> 105
<211> 9
<212> PRT
<213> Homo sapiens

<400> 105

Gln Gln Tyr Tyr Arg Phe Pro Leu Thr
1               5

<210> 106
<211> 13
<212> PRT

<213> Homo sapiens

<400> 106

Ser Gly Arg Ser Ser Asn Ile Glu Ser Asn Asn Val Asn
1               5                   10

<210> 107
<211> 7
<212> PRT
<213> Homo sapiens

<400> 107

Ser Asn Asn Gln Arg Pro Ser
1               5

<210> 108
<211> 11
<212> PRT
<213> Homo sapiens

<400> 108

Ala Ala Trp Asp Asp Ser Leu Ser Gly Gln Val
1               5                   10

<210> 109
<211> 17

<212> PRT
<213> Homo sapiens

<400> 109

Lys Ser Ser Gln Ser Val Leu Tyr Ser Ser Asn Lys Lys Asn Tyr Leu
1               5                   10                  15
Ala

<210> 110
<211> 7
<212> PRT
<213> Homo sapiens

<400> 110

Trp Ala Ser Thr Arg Glu Ser
1               5

<210> 111
<211> 9
<212> PRT
<213> Homo sapiens

<400> 111

Gln Gln Tyr Tyr Ser Thr Pro Leu Thr
1               5

<210> 112
<211> 13
<212> PRT
<213> Homo sapiens

<400> 112

Ser Gly Ser Ser Ser Asn Ile Gly Asn Asn Tyr Val Ser
1               5                   10

<210> 113
<211> 7
<212> PRT
<213> Homo sapiens

<400> 113

Asp Asn Asn Lys Arg Pro Ser
1               5

<210> 114
<211> 11
<212> PRT
<213> Homo sapiens

<400> 114

Gly Thr Trp Asp Ser Ser Leu Ser Ala Gly Val
1               5                   10

<210>  115
<211>  11
<212>  PRT
<213>  Homo sapiens

<400>  115

Ser Gly Asp Lys Leu Gly Asp Lys Tyr Ala Cys
1               5                   10

<210>  116
<211>  7
<212>  PRT
<213>  Homo sapiens

<400>  116

Gln Asp Ser Lys Arg Pro Ser
1               5

<210>  117
<211>  9
<212>  PRT
<213>  Homo sapiens

<400>  117

Gln Ala Trp Asp Ser Ser Thr Tyr Val
1               5

<210>  118
<211>  11
<212>  PRT
<213>  Homo sapiens

<400>  118

Gly Gly Asn Asn Ile Gly Ser Lys Asn Val His
1               5                   10

<210>  119
<211>  7
<212>  PRT
<213>  Homo sapiens

<400>  119

Arg Asp Ser Asn Arg Pro Ser
1               5

<210>  120
<211>  11
<212>  PRT
<213>  Homo sapiens

<400> 120

Gln Val Trp Asp Ser Ser Thr Ala Cys Gly Val
1               5                   10

<210> 121
<211> 11
<212> PRT
<213> Homo sapiens

<400> 121

Gln Gly Asp Ser Leu Arg Ser Tyr Tyr Ala Ser
1               5                   10

<210> 122
<211> 7
<212> PRT
<213> Homo sapiens

<400> 122

Gly Lys Asn Asn Arg Pro Ser
1               5

<210> 123
<211> 11
<212> PRT
<213> Homo sapiens

<400> 123

Asn Ser Arg Asp Ser Ser Gly Asn His Leu Val
1               5                   10

<210> 124
<211> 14
<212> PRT
<213> Homo sapiens

<400> 124

Thr Gly Thr Ser Ser Asp Val Gly Gly Tyr Asn Tyr Val Ser
1               5                   10

<210> 125
<211> 7
<212> PRT
<213> Homo sapiens

<400> 125

Glu Val Thr Lys Arg Pro Ser
1               5

<210> 126
<211> 10
<212> PRT

<213> Homo sapiens

<400> 126

Cys Ser Tyr Ala Gly Ser Tyr Thr Phe Leu
1               5                   10

<210> 127
<211> 11
<212> PRT
<213> Homo sapiens

<400> 127

Arg Ala Ser Gln Ser Ile Ser Ser Trp Leu Ala
1               5                   10

<210> 128
<211> 7
<212> PRT
<213> Homo sapiens

<400> 128

Lys Ala Ser Ser Leu Glu Ser
1               5

<210> 129
<211> 9

<212> PRT
<213> Homo sapiens

<400> 129

Gln Gln Tyr Asn Ser Tyr Pro Tyr Thr
1               5

<210> 130
<211> 14
<212> PRT
<213> Homo sapiens

<400> 130

Thr Gly Thr Ser Ser Asp Val Gly Gly Tyr Asn Tyr Val Ser
1               5                   10

<210> 131
<211> 7
<212> PRT
<213> Homo sapiens

<400> 131

Glu Val Ser Lys Arg Pro Ser
1               5

<210> 132

<211> 11
<212> PRT
<213> Homo sapiens

<400> 132

Ser Ser Tyr Ala Gly Ser Asn Asn Leu Tyr Val
1               5                   10

<210> 133
<211> 11
<212> PRT
<213> Homo sapiens

<400> 133

Arg Ala Ser Gln Ser Val Ser Arg Tyr Leu Ala
1               5                   10

<210> 134
<211> 7
<212> PRT
<213> Homo sapiens

<400> 134

Asp Ala Ser Asn Arg Ala Thr
1               5

<210> 135
<211> 10
<212> PRT
<213> Homo sapiens

<400> 135

Gln Gln Arg Ser His Trp Gln Pro Leu Thr
1               5                   10

<210> 136
<211> 13
<212> PRT
<213> Homo sapiens

<400> 136

Ser Gly Ser Ser Ser Asn Ile Gly Asn Asn Tyr Val Ser
1               5                   10

<210> 137
<211> 7
<212> PRT
<213> Homo sapiens

<400> 137

Asp Asn Asn Lys Arg Pro Ser
1               5

<210> 138
<211> 11
<212> PRT
<213> Homo sapiens

<400> 138

Gly Thr Trp Asp Ser Ser Leu Ser Ala Tyr Val
1               5                   10

<210> 139
<211> 11
<212> PRT
<213> Homo sapiens

<400> 139

Arg Ala Ser Gln Ser Ile Ser Ser Tyr Leu Asn
1               5                   10

<210> 140
<211> 7
<212> PRT
<213> Homo sapiens

<400> 140

Ala Ala Ser Ser Leu Gln Ser

1               5

<210> 141
<211> 9
<212> PRT
<213> Homo sapiens

<400> 141

Gln Gln Ser Tyr Ser Thr Pro Leu Thr
1               5

<210> 142
<211> 17
<212> PRT
<213> homo sapiens

<400> 142

Lys Ser Ser Gln Ser Val Leu Tyr Ser Ser Asn Asn Lys Asp Tyr Phe
1               5                   10                  15
Ala

<210> 143
<211> 140
<212> PRT
<213> homo sapiens

<400> 143

Met Glu Phe Gly Leu Arg Trp Leu Phe Leu Val Ala Phe Leu Lys Gly
1               5                   10                  15

Val Gln Cys Glu Val Gln Leu Leu Glu Ser Gly Gly Asp Leu Val Gln
            20                  25                  30

Pro Gly Gly Ser Leu Arg Leu Ser Cys Ala Ala Ser Gly Phe Thr Phe
        35                  40                  45

Ser Asn Tyr Ala Met Ala Trp Val Arg Gln Ala Pro Gly Lys Gly Leu
    50                  55                  60

Glu Trp Val Ser Ala Ile Ser Val Ser Gly Thr Ser Thr Ala Tyr Ala
65                  70                  75                  80

Asp Ser Val Lys Gly Arg Phe Thr Val Ser Arg Asp Asn Ser Lys Asn
                85                  90                  95

Thr Leu Tyr Leu Gln Met Asn Ser Leu Arg Ala Asp Asp Thr Ala Val
            100                 105                 110

Tyr Tyr Cys Ala Lys Arg Gly Leu Pro His Pro Lys Tyr Phe Cys Asp
        115                 120                 125

Ser Trp Gly Gln Gly Thr Met Val Thr Val Ser Ser
    130                 135                 140

<210> 144
<211> 129
<212> PRT
<213> homo sapiens

<400> 144

Met Ala Trp Ala Leu Leu Phe Leu Thr Leu Leu Thr Gln Gly Thr Gly

1               5                   10                  15

Ser Trp Ala Gln Ser Ala Leu Thr Gln Pro Ala Ser Val Ser Gly Ser

            20                  25                  30

Pro Gly Gln Ser Ile Thr Ile Ser Cys Thr Gly Thr Ser Ser Asp Ile

        35                  40                  45

Gly Ala Tyr Asp Phe Val Ser Trp Tyr Gln Gln His Pro Gly Lys Ala

    50                  55                  60

Pro Glu Leu Val Ile Tyr Asp Val Arg Asn Arg Pro Ser Gly Val Ser

65                  70                  75                  80

Asn Arg Phe Ser Ala Ser Lys Ser Gly Asn Thr Ala Ser Leu Thr Ile

                85                  90                  95

Ser Gly Leu Gln Ala Glu Asp Glu Ala Asp Tyr Tyr Cys Ser Ser Phe

            100                 105                 110

Thr Thr Ser Ser Val Trp Val Phe Gly Gly Gly Thr Lys Leu Thr Val

        115                 120                 125

Leu

<210>  145
<211>  421
<212>  DNA
<213>  homo sapiens

<400>  145
atggagtttg ggctgaggtg gctttttctt gtggcttttt taaaaggtgt ccagtgcgag      60
gtacagctgt tggagtctgg gggagacttg gtacagcctg gggggtccct gagactctcc     120
tgtgcagcct ctggattcac ctttagtaac tatgccatgg cctgggtccg ccaggctcca     180
gggaaggggc tggagtgggt ctcagcaatt agtgttagtg gtactagcac agcctacgca     240

gactccgtga agggccggtt caccgtctcc agagacaatt ccaagaacac gctgtattta 300

caaatgaaca gcctgagagc cgacgacacg gccgtatatt actgtgcgaa aagagggcta 360

ccgcacccga aatacttctg tgactcctgg ggccagggaa ccatggtcac cgtctcctca 420

g 421

<210> 146
<211> 387
<212> DNA
<213> homo sapiens

<400> 146
atggcctggg ctctgctatt cctcaccctc ctcactcagg gcacagggtc ctgggcccag 60

tctgccctga ctcagcctgc ctccgtgtct gggtctcctg gacagtcgat caccatctcc 120

tgcactggaa ccagcagtga cattggtgct tatgactttg tctcctggta ccaacaacac 180

ccaggcaaag cccccgaact cgtgatttat gatgtccgta atcggccctc aggggtttct 240

aatcgcttct ctgcctccaa gtctggcaac acggcctccc tgaccatctc tgggctccag 300

gctgaggacg aggctgatta ttactgcagc tcatttacaa ccagcagcgt ttgggtgttc 360

ggcggaggga ccaagctgac cgtccta 387

<210> 147
<211> 15
<212> DNA
<213> homo sapiens

<400> 147
aactatgcca tggcc 15

<210> 148

<211>  51
<212>  DNA
<213>  homo sapiens

<400>  148
gcaattagtg ttagtggtac tagcacagcc tacgcagact ccgtgaaggg c          51

<210>  149
<211>  36
<212>  DNA
<213>  homo sapiens

<400>  149
agagggctac cgcacccgaa atacttctgt gactcc          36

<210>  150
<211>  42
<212>  DNA
<213>  homo sapiens

<400>  150
actggaacca gcagtgacat tggtgcttat gactttgtct cc          42

<210>  151
<211>  21
<212>  DNA
<213>  homo sapiens

<400>  151

gatgtccgta atcggccctc a 21

<210> 152
<211> 30
<212> DNA
<213> homo sapiens

<400> 152
agctcattta caaccagcag cgtttgggtg 30

<210> 153
<211> 22
<212> DNA
<213> Artificial

<220>
<223> Primer

<400> 153
ggagtttcca ttcggtgatc ag 22

<210> 154
<211> 22
<212> DNA
<213> Artificial

<220>
<223> Primer

<400> 154
gtgctggggt ctcaggaggc ag 22

<210> 155
<211> 21
<212> DNA
<213> Artificial

<220>
<223> Primer

<400> 155
ctatgaacat tctgtagggg c 21

<210> 156
<211> 21
<212> DNA
<213> Artificial

<220>
<223> Primer

<400> 156
tgcagctcta gtctcccgtg g 21

<210> 157
<211> 20
<212> DNA
<213> Artificial

<220>
<223> Primer

<400> 157
gggaaggaag tcctgtgcga 20

<210> 158
<211> 22
<212> DNA
<213> Artificial

<220>
<223> Primer

<400> 158
cagctgtgag cgcagaaggc ag 22

<210> 159
<211> 20
<212> DNA
<213> Artificial

<220>
<223> Primer

<400> 159
tcgggacaat cttcatcatg 20

<210> 160
<211> 21
<212> DNA
<213> Artificial

<220>
<223> Primer

<400> 160
cggtcacatg gcaccacctc t 21

<210> 161
<211> 30
<212> DNA
<213> Artificial

<220>
<223> Primer

<400> 161
tggacaagag agttgagtcc aaatatggtc 30

<210> 162
<211> 30
<212> DNA
<213> Artificial

<220>
<223> Primer

<400> 162
gaccatattt ggactcaact ctcttgtcca 30

<210> 163
<211> 30
<212> DNA
<213> Artificial

<220>
<223> Primer

<400> 163
gcccatcatg cccagcacct gagttcctgg 30

<210> 164
<211> 30
<212> DNA
<213> Artificial

<220>
<223> Primer

<400> 164
ccaggaactc aggtgctggg catgatgggc 30

<210> 165
<211> 30
<212> DNA

<213>  Artificial

<220>
<223>  Primer

<400>  165
tctccaaagc caaagggcag ccccgagagc                                        30

<210>  166
<211>  30
<212>  DNA
<213>  Artificial

<220>
<223>  Primer

<400>  166
gctctcgggg ctgccctttg gctttggaga                                        30

<210>  167
<211>  21
<212>  DNA
<213>  Artificial

<220>
<223>  Primer

<400>  167
ccctggcact catttaccca g                                                 21

<210> 168
<211> 24
<212> DNA
<213> Artificial

<220>
<223> Primer

<400> 168
gacggggtac gtgccaagca tcct 24

<210> 169
<211> 30
<212> DNA
<213> Artificial

<220>
<223> Primer

<400> 169
agcgctagca ccaagggccc atccgtcttc 30

<210> 170
<211> 30
<212> DNA
<213> Artificial

<220>

<223>  Primer

<400>  170
agatcttcat  gggggaccat  atttggactc                                    30

<210>  171
<211>  30
<212>  DNA
<213>  Artificial

<220>
<223>  Primer

<400>  171
ttaatgatga  tgatgatgat  gtgggggacc                                    30

<210>  172
<211>  18
<212>  DNA
<213>  Artificial

<220>
<223>  Primer

<400>  172
gttttcccag  tcacgacg                                                  18

<210>  173
<211>  25

<212>  DNA
<213>  Artificial

<220>
<223>  Primer

<400>  173
gggcggagta ctggagacgg tgacc                                          25

<210>  174
<211>  27
<212>  DNA
<213>  Artificial

<220>
<223>  Primer

<400>  174
cccccgctag cgctggagac ggtgacc                                        27

<210>  175
<211>  30
<212>  DNA
<213>  Artificial

<220>
<223>  Primer

<400>  175

ggatccaaaa caacagcccc atcggtctat 30

<210> 176
<211> 30
<212> DNA
<213> Artificial

<220>
<223> Primer

<400> 176
tggacaagaa aattgagccc agagtgccca 30

<210> 177
<211> 30
<212> DNA
<213> Artificial

<220>
<223> Primer

<400> 177
tgggcactct gggctcaatt ttcttgtcca 30

<210> 178
<211> 30
<212> DNA
<213> Artificial

<220>
<223> Primer

<400> 178
gtcccccatg cgcagctcca gacctcttgg 30

<210> 179
<211> 30
<212> DNA
<213> Artificial

<220>
<223> Primer

<400> 179
ccaagaggtc tggagctgcg catgggggac 30

<210> 180
<211> 30
<212> DNA
<213> Artificial

<220>
<223> Primer

<400> 180
tctcaaaacc cagagggcca gtaagagctc 30

<210> 181

<211> 30
<212> DNA
<213> Artificial

<220>
<223> Primer

<400> 181
gagctcttac tggccctctg ggttttgaga 30

<210> 182
<211> 30
<212> DNA
<213> Artificial

<220>
<223> Primer

<400> 182
agatcttcat ttacccagag accgggagat 30

## Claims

1. A human antibody that specifically binds to human platelet membrane glycoprotein VI and does not induce a human platelet agglutination independently, or an active-fragment thereof.

2. A human antibody that specifically binds to human platelet membrane glycoprotein VI and suppresses collagen-mediated human platelet agglutination by in vivo administration, or an active-fragment thereof.

3. A human antibody that specifically binds to human GPVI and has any one or more functions of the following functions: specifically inhibiting a binding of GPVI on the platelet to collagen; disappearing a functionable GPVI on the platelet; or decreasing or deleting a collagen-mediated agglutinability of the human platelet by preliminarily contacting with the human platelet, or an active-fragment thereof.

4. A human antibody that specifically binds to human platelet membrane glycoprotein VI, suppresses a collagen-mediated agglutinability of the human platelet and does not induce a human platelet agglutination independently, or an active-fragment thereof.

5. An antibody or an active fragment thereof, wherein VH CDR1, VH CDR2, VHCDR3, VLCDR1, VLCDR2, and VLCDR3 has the amino acid sequence of SEQ ID NO: 47, SEQ ID NO: 48, SEQ ID NO: 49, SEQ ID NO: 98, SEQ ID NO: 99 and SEQ ID NO: 100, respectively; or VH CDR1, VH CDR2, VHCDR3, VLCDR1, VLCDR2, and VLCDR3 has the amino acid sequence of SEQ ID NO: 7, SEQ ID NO: 8, SEQ ID NO: 9, SEQ ID NO: 10, SEQ ID NO: 11 and SEQ ID NO: 12, respectively.

6. A cell that produces the antibody of claims 1 through 5 or an active fragment thereof

7. A polynucleotide that comprises a base sequence encoding a H chain and/or L chain of the antibody of claims 1 through 5 or an active fragment thereof

8. A pharmaceutical composition that comprises the antibody of claims 1 through 4 or an active fragment thereof as an active ingredient.

9. A method for producing a recombinant human-human chimeric antibody
